# EUROPEAN PATENT APPLICATION

(11) **EP 2 471 547 A1**
(43) Date of publication of application: **04.07.2012**
(21) Application number: 10812094.0
(22) Date of filing: 25.08.2010
(51) Int. Cl.: A61K 38/00, A61P 35/00, A61P 43/00, C07K 14/485, C07K 14/745

(54) **APOPTOSIS INDUCER**

(30) Priority: 25.08.2009 JP 2009194747
(71) Applicant: Nihon University, Tokyo 102-8275 (JP)
(72) Inventor: HIDAI, Chiaki, Tokyo 102-8275 (JP); KITANO, Hisataka, Tokyo 102-8275 (JP)
(74) Representative: Denison, Christopher Marcus
(86) International application number: PCT/JP2010/064857
(87) International publication number: WO 2011/025050

(57) **Abstract**

The present invention provides an apoptosis-inducing agent capable of inducing apoptosis in various cells including tumor cells and other cells in a simple manner, etc. The apoptosis-inducing agent of the present invention comprises a peptide shown in (a) or (b) below, a derivative thereof, or a salt of the peptide or the derivative: (a) a peptide which comprises an amino acid sequence represented by the formula: C-X-D-X-X-X-X-Y-X-C-X-C (I); or (b) a peptide which comprises an amino acid sequence with deletion, substitution or addition of one or several amino acids in the amino acid sequence represented by formula (I) and which has apoptosis-inducing activity.

SEQUENCE LISTING

<110>  NIHON UNIVERSITY

<120>  apoptosis-inducing agent

<130>  CMD/FP6806996

<140>  10812094.0
<141>  2010-08-25

<150>  PCT/JP2010/064857
<151>  2010-08-25

<150>  JP 2009-194747
<151>  2009-08-25

<160>  44

<170>  PatentIn version 3.3

<210>  1
<211>  12
<212>  PRT
<213>  Artificial

<220>
<223>  peptide


<220>
<221>  MISC_FEATURE
<222>  (2)..(2)
<223>  Xaa can be any naturally occurring amino acid

<220>
<221>  MISC_FEATURE
<222>  (4)..(7)
<223>  Xaa can be any naturally occurring amino acid

<220>
<221>  MISC_FEATURE
<222>  (9)..(9)
<223>  Xaa can be any naturally occurring amino acid

<220>
<221>  MISC_FEATURE
<222>  (11)..(11)
<223>  Xaa can be any naturally occurring amino acid

<400>  1

Cys Xaa Asp Xaa Xaa Xaa Xaa Tyr Xaa Cys Xaa Cys
1               5                   10


<210>  2
<211>  12
<212>  PRT
<213>  Mus musculus

<400>  2

Cys Thr Asp Leu Val Ala Asn Tyr Ser Cys Glu Cys
1               5                   10

<210>  3
<211>  12
<212>  PRT
<213>  Mus musculus

<400>  3

Cys Thr Asp Leu Val Ala Asn Tyr Ser Cys Glu Cys
1               5                   10


<210>  4
<211>  2303
<212>  DNA
<213>  Mus musculus


<220>
<221>  CDS
<222>  (619)..(2061)

<400>  4
gaattccggt taactgagga caaagggtaa tgcagaagtg atatttgatt tccattctca      60

ttcccagtgg ccttgatatt taaactgatt cctgccacca ggtccttggg ccaccctgtc     120

cctgcgtctc atatttctgc atgctgcttt gtttgtatat agtgcgctcc tggcctcagg     180

ctcgctcccc tccagctctc gcttcattgt tctccaagtc agaagccccc gcatccgccg     240

cgcagcagcg tgagccgtag tcactgctgg ccgcttcgcc tgcgtgcgcg cacggaaatc     300

ggggagccag gaacccaagg agccgccgtc cgcccgctgt gcctctgcta gaccactcgc     360

agccccagcc tctctcaagc gcacccacct ccgcgcaccc cagctcaggc gaagctggag     420

tgagggtgaa tcaccctttc tctagggcca ccactctttt atcgcccttc ccaagatttg     480

agaagcgctg cgggaggaaa gacgtcctct tgatctctga cagggcgggg tttactgctg     540

tcctgcaggc gcgcctcgcc tactgtgccc tccgctacga ccccggacca gcccaggtca     600

cgtccgtgag aagggatc atg aag cac ttg gta gca gcc tgg ctt ttg gtt       651
                    Met Lys His Leu Val Ala Ala Trp Leu Leu Val
                    1               5                   10

gga ctc agc ctc ggg gtg ccc cag ttc ggc aaa ggt gac att tgc aac       699
Gly Leu Ser Leu Gly Val Pro Gln Phe Gly Lys Gly Asp Ile Cys Asn
            15                  20                  25

ccg aac ccc tgt gaa aat ggt ggc atc tgt ctg tca gga ctg gct gat       747
Pro Asn Pro Cys Glu Asn Gly Gly Ile Cys Leu Ser Gly Leu Ala Asp
        30                  35                  40

gat tcc ttt tcc tgt gag tgt cca gaa ggc ttc gca ggt ccg aac tgc       795
Asp Ser Phe Ser Cys Glu Cys Pro Glu Gly Phe Ala Gly Pro Asn Cys
    45                  50                  55

tct agt gtt gtg gag gtt gca tca gat gaa gaa aag cct act tca gca       843
Ser Ser Val Val Glu Val Ala Ser Asp Glu Glu Lys Pro Thr Ser Ala
60                  65                  70                  75

ggt ccc tgc atc cct aac cca tgc cat aac gga gga acc tgt gag ata       891

Gly Pro Cys Ile Pro Asn Pro Cys His Asn Gly Gly Thr Cys Glu Ile
                80                  85                  90

agc gaa gcc tat cga gga gac aca ttc ata ggc tat gtt tgt aaa tgt 939
Ser Glu Ala Tyr Arg Gly Asp Thr Phe Ile Gly Tyr Val Cys Lys Cys
            95                  100                 105

cct cgg gga ttt aat ggg att cac tgt cag cac aat ata aat gaa tgt 987
Pro Arg Gly Phe Asn Gly Ile His Cys Gln His Asn Ile Asn Glu Cys
        110                 115                 120

gaa gct gag cct tgc aga aat ggc gga ata tgt acc gac ctt gtt gct 1035
Glu Ala Glu Pro Cys Arg Asn Gly Gly Ile Cys Thr Asp Leu Val Ala
    125                 130                 135

aac tac tct tgt gaa tgc cca gga gaa ttt atg gga cga aat tgt caa 1083
Asn Tyr Ser Cys Glu Cys Pro Gly Glu Phe Met Gly Arg Asn Cys Gln
140                 145                 150                 155

tat aaa tgc tct ggg cca ttg gga atc gaa ggt ggg atc ata tct aat 1131
Tyr Lys Cys Ser Gly Pro Leu Gly Ile Glu Gly Gly Ile Ile Ser Asn
                160                 165                 170

cag caa atc aca gct tca tct act cac cga gct ctt ttt gga ctc cgg 1179
Gln Gln Ile Thr Ala Ser Ser Thr His Arg Ala Leu Phe Gly Leu Arg
            175                 180                 185

aag tgg tat ccc tac tat gct cga ctt aat aag aag ggc ctt ata aat 1227
Lys Trp Tyr Pro Tyr Tyr Ala Arg Leu Asn Lys Lys Gly Leu Ile Asn
        190                 195                 200

gcc tgg aca gct gct gaa aat gac aga tgg cca tgg att cag ata aat 1275
Ala Trp Thr Ala Ala Glu Asn Asp Arg Trp Pro Trp Ile Gln Ile Asn
    205                 210                 215

ttg caa aga aaa atg aga gtc act ggt gtt att acc caa gga gca aaa 1323
Leu Gln Arg Lys Met Arg Val Thr Gly Val Ile Thr Gln Gly Ala Lys
220                 225                 230                 235

agg att gga agc cca gag tac ata aaa tcc tac aaa att gcc tac agc 1371
Arg Ile Gly Ser Pro Glu Tyr Ile Lys Ser Tyr Lys Ile Ala Tyr Ser
                240                 245                 250

aat gac ggg aag acc tgg gca atg tac aaa gta aaa ggc acc aat gaa 1419
Asn Asp Gly Lys Thr Trp Ala Met Tyr Lys Val Lys Gly Thr Asn Glu
            255                 260                 265

gag atg gtc ttt cgt gga aat gtt gat aac aac aca cca tat gct aat 1467
Glu Met Val Phe Arg Gly Asn Val Asp Asn Asn Thr Pro Tyr Ala Asn
        270                 275                 280

tct ttc aca ccc cca atc aaa gct cag tat gta aga ctc tac ccc caa 1515
Ser Phe Thr Pro Pro Ile Lys Ala Gln Tyr Val Arg Leu Tyr Pro Gln
    285                 290                 295

att tgt cga agg cat tgt act tta aga atg gaa ctt ctt ggc tgt gag 1563
Ile Cys Arg Arg His Cys Thr Leu Arg Met Glu Leu Leu Gly Cys Glu
300                 305                 310                 315

ctc tca ggc tgt tca gaa cct ttg ggg atg aaa tca ggg cat ata caa 1611
Leu Ser Gly Cys Ser Glu Pro Leu Gly Met Lys Ser Gly His Ile Gln
                320                 325                 330

gac tac cag atc act gcc tcc agc gtc ttc aga aca ctc aac atg gac 1659
Asp Tyr Gln Ile Thr Ala Ser Ser Val Phe Arg Thr Leu Asn Met Asp

335                 340                 345

atg ttt act tgg gaa cca agg aaa gcc agg ctg gac aag caa ggc aaa      1707
Met Phe Thr Trp Glu Pro Arg Lys Ala Arg Leu Asp Lys Gln Gly Lys
        350                 355                 360

gta aat gcc tgg act tcc ggc cat aac gac cag tca caa tgg tta cag      1755
Val Asn Ala Trp Thr Ser Gly His Asn Asp Gln Ser Gln Trp Leu Gln
    365                 370                 375

gtt gat ctt ctt gtc cct act aag gtg aca ggc atc att aca caa gga      1803
Val Asp Leu Leu Val Pro Thr Lys Val Thr Gly Ile Ile Thr Gln Gly
380                 385                 390                 395

gct aaa gat ttt ggt cac gtg cag ttt gtt ggg tca tac aaa cta gct      1851
Ala Lys Asp Phe Gly His Val Gln Phe Val Gly Ser Tyr Lys Leu Ala
                400                 405                 410

tac agc aat gat gga gaa cac tgg atg gtg cac cag gat gaa aaa cag      1899
Tyr Ser Asn Asp Gly Glu His Trp Met Val His Gln Asp Glu Lys Gln
            415                 420                 425

agg aaa gac aag gtt ttt caa ggc aat ttt gac aat gac act cac agg      1947
Arg Lys Asp Lys Val Phe Gln Gly Asn Phe Asp Asn Asp Thr His Arg
        430                 435                 440

aaa aat gtc atc gac cct ccc atc tat gca cga ttc ata aga atc ctt      1995
Lys Asn Val Ile Asp Pro Pro Ile Tyr Ala Arg Phe Ile Arg Ile Leu
    445                 450                 455

cct tgg tcc tgg tat gga agg atc act ctg cgg tca gag ctg ctg ggc      2043
Pro Trp Ser Trp Tyr Gly Arg Ile Thr Leu Arg Ser Glu Leu Leu Gly
460                 465                 470                 475

tgc gca gag gag gaa tga agtgcggggc cgcacatccc acaatgcttt             2091
Cys Ala Glu Glu Glu
                480

tctttatttt cctataagta tctccacgaa atgaactgtg tgaagctgat ggaaactgca    2151

tttgtttttt tcaaagtgtt caaattatgg taggctactg actgtctttt taggagttct    2211

aagcttgcct ttttaataat ttaatttggt ttcctttgct caactctctt atgtaatatc     2271

acactgtctg tgagttactc ttcttgttct ct                                  2303


<210>  5
<211>  480
<212>  PRT
<213>  Mus musculus

<400>  5

Met Lys His Leu Val Ala Ala Trp Leu Leu Val Gly Leu Ser Leu Gly
1               5                   10                  15


Val Pro Gln Phe Gly Lys Gly Asp Ile Cys Asn Pro Asn Pro Cys Glu
            20                  25                  30


Asn Gly Gly Ile Cys Leu Ser Gly Leu Ala Asp Asp Ser Phe Ser Cys
        35                  40                  45

Glu Cys Pro Glu Gly Phe Ala Gly Pro Asn Cys Ser Ser Val Val Glu
    50                  55                  60

Val Ala Ser Asp Glu Glu Lys Pro Thr Ser Ala Gly Pro Cys Ile Pro
65                  70                  75                  80

Asn Pro Cys His Asn Gly Gly Thr Cys Glu Ile Ser Glu Ala Tyr Arg
                85                  90                  95

Gly Asp Thr Phe Ile Gly Tyr Val Cys Lys Cys Pro Arg Gly Phe Asn
            100                 105                 110

Gly Ile His Cys Gln His Asn Ile Asn Glu Cys Glu Ala Glu Pro Cys
        115                 120                 125

Arg Asn Gly Gly Ile Cys Thr Asp Leu Val Ala Asn Tyr Ser Cys Glu
    130                 135                 140

Cys Pro Gly Glu Phe Met Gly Arg Asn Cys Gln Tyr Lys Cys Ser Gly
145                 150                 155                 160

Pro Leu Gly Ile Glu Gly Gly Ile Ile Ser Asn Gln Gln Ile Thr Ala
                165                 170                 175

Ser Ser Thr His Arg Ala Leu Phe Gly Leu Arg Lys Trp Tyr Pro Tyr
            180                 185                 190

Tyr Ala Arg Leu Asn Lys Lys Gly Leu Ile Asn Ala Trp Thr Ala Ala
        195                 200                 205

Glu Asn Asp Arg Trp Pro Trp Ile Gln Ile Asn Leu Gln Arg Lys Met
    210                 215                 220

Arg Val Thr Gly Val Ile Thr Gln Gly Ala Lys Arg Ile Gly Ser Pro
225                 230                 235                 240

Glu Tyr Ile Lys Ser Tyr Lys Ile Ala Tyr Ser Asn Asp Gly Lys Thr
                245                 250                 255

Trp Ala Met Tyr Lys Val Lys Gly Thr Asn Glu Glu Met Val Phe Arg
            260                 265                 270

Gly Asn Val Asp Asn Asn Thr Pro Tyr Ala Asn Ser Phe Thr Pro Pro
        275                 280                 285

Ile Lys Ala Gln Tyr Val Arg Leu Tyr Pro Gln Ile Cys Arg Arg His
    290                 295                 300

Cys Thr Leu Arg Met Glu Leu Leu Gly Cys Glu Leu Ser Gly Cys Ser
305                 310                 315                 320

Glu Pro Leu Gly Met Lys Ser Gly His Ile Gln Asp Tyr Gln Ile Thr
                325                 330                 335

Ala Ser Ser Val Phe Arg Thr Leu Asn Met Asp Met Phe Thr Trp Glu
            340                 345                 350

Pro Arg Lys Ala Arg Leu Asp Lys Gln Gly Lys Val Asn Ala Trp Thr
        355                 360                 365

Ser Gly His Asn Asp Gln Ser Gln Trp Leu Gln Val Asp Leu Leu Val
    370                 375                 380

Pro Thr Lys Val Thr Gly Ile Ile Thr Gln Gly Ala Lys Asp Phe Gly
385                 390                 395                 400

His Val Gln Phe Val Gly Ser Tyr Lys Leu Ala Tyr Ser Asn Asp Gly
                405                 410                 415

Glu His Trp Met Val His Gln Asp Glu Lys Gln Arg Lys Asp Lys Val
            420                 425                 430

Phe Gln Gly Asn Phe Asp Asn Asp Thr His Arg Lys Asn Val Ile Asp
        435                 440                 445

Pro Pro Ile Tyr Ala Arg Phe Ile Arg Ile Leu Pro Trp Ser Trp Tyr
    450                 455                 460

Gly Arg Ile Thr Leu Arg Ser Glu Leu Leu Gly Cys Ala Glu Glu Glu
465                 470                 475                 480


<210>  6
<211>  105
<212>  DNA
<213>  Mus musculus


<220>
<221>  CDS
<222>  (1)..(105)

<400>  6
tgt gaa gct gag cct tgc aga aat ggc gga ata tgt acc gac ctt gtt       48
Cys Glu Ala Glu Pro Cys Arg Asn Gly Gly Ile Cys Thr Asp Leu Val
1               5                   10                  15

gct aac tac tct tgt gaa tgc cca gga gaa ttt atg gga cga aat tgt       96
Ala Asn Tyr Ser Cys Glu Cys Pro Gly Glu Phe Met Gly Arg Asn Cys
            20                  25                  30

caa tat aaa                                                           105
Gln Tyr Lys

        35

<210>  7
<211>  35
<212>  PRT
<213>  Mus musculus

<400>  7

Cys Glu Ala Glu Pro Cys Arg Asn Gly Gly Ile Cys Thr Asp Leu Val
1               5                   10                  15

Ala Asn Tyr Ser Cys Glu Cys Pro Gly Glu Phe Met Gly Arg Asn Cys
            20                  25                  30

Gln Tyr Lys
        35

<210>  8
<211>  1044
<212>  DNA
<213>  Mus musculus

<220>
<221>  CDS
<222>  (1)..(1044)

<400>  8
atg aag cac ttg gta gca gcc tgg ctt ttg gtt gga ctc agc ctc ggg      48
Met Lys His Leu Val Ala Ala Trp Leu Leu Val Gly Leu Ser Leu Gly
1               5                   10                  15

gtg ccc cag ttc ggc aaa ggt gac att tgc aac ccg aac ccc tgt gaa      96
Val Pro Gln Phe Gly Lys Gly Asp Ile Cys Asn Pro Asn Pro Cys Glu
            20                  25                  30

aat ggt ggc atc tgt ctg tca gga ctg gct gat gat tcc ttt tcc tgt      144
Asn Gly Gly Ile Cys Leu Ser Gly Leu Ala Asp Asp Ser Phe Ser Cys
        35                  40                  45

gag tgt cca gaa ggc ttc gca ggt ccg aac tgc tct agt gtt gtg gag      192
Glu Cys Pro Glu Gly Phe Ala Gly Pro Asn Cys Ser Ser Val Val Glu
    50                  55                  60

gtt gca tca gat gaa gaa aag cct act tca gca ggt ccc tgc atc cct      240
Val Ala Ser Asp Glu Glu Lys Pro Thr Ser Ala Gly Pro Cys Ile Pro
65                  70                  75                  80

aac cca tgc cat aac gga gga acc tgt gag ata agc gaa gcc tat cga      288
Asn Pro Cys His Asn Gly Gly Thr Cys Glu Ile Ser Glu Ala Tyr Arg
                85                  90                  95

gga gac aca ttc ata ggc tat gtt tgt aaa tgt cct cgg gga ttt aat      336
Gly Asp Thr Phe Ile Gly Tyr Val Cys Lys Cys Pro Arg Gly Phe Asn
            100                 105                 110

ggg att cac tgt cag cac aat ata aat gaa tgt gaa gct gag cct tgc      384
Gly Ile His Cys Gln His Asn Ile Asn Glu Cys Glu Ala Glu Pro Cys
        115                 120                 125

aga aat ggc gga ata tgt acc gac ctt gtt gct aac tac tct tgt gaa      432
Arg Asn Gly Gly Ile Cys Thr Asp Leu Val Ala Asn Tyr Ser Cys Glu
    130                 135                 140

tgc cca gga gaa ttt atg gga cga aat tgt caa tat aaa tgc tct ggg      480
Cys Pro Gly Glu Phe Met Gly Arg Asn Cys Gln Tyr Lys Cys Ser Gly
145                 150                 155                 160

cca ttg gga atc gaa ggt ggg atc ata tct aat cag caa atc aca gct      528
Pro Leu Gly Ile Glu Gly Gly Ile Ile Ser Asn Gln Gln Ile Thr Ala
                165                 170                 175

tca tct act cac cga gct ctt ttt gga ctc cgg aag tgg tat ccc tac      576
Ser Ser Thr His Arg Ala Leu Phe Gly Leu Arg Lys Trp Tyr Pro Tyr
            180                 185                 190

tat gct cga ctt aat aag aag ggc ctt ata aat gcc tgg aca gct gct      624
Tyr Ala Arg Leu Asn Lys Lys Gly Leu Ile Asn Ala Trp Thr Ala Ala
        195                 200                 205

gaa aat gac aga tgg cca tgg att cag ata aat ttg caa aga aaa atg      672
Glu Asn Asp Arg Trp Pro Trp Ile Gln Ile Asn Leu Gln Arg Lys Met
    210                 215                 220

aga gtc act ggt gtt att acc caa gga gca aaa agg att gga agc cca      720
Arg Val Thr Gly Val Ile Thr Gln Gly Ala Lys Arg Ile Gly Ser Pro
225                 230                 235                 240

gag tac ata aaa tcc tac aaa att gcc tac agc aat gac ggg aag acc      768
Glu Tyr Ile Lys Ser Tyr Lys Ile Ala Tyr Ser Asn Asp Gly Lys Thr
                245                 250                 255

tgg gca atg tac aaa gta aaa ggc acc aat gaa gag atg gtc ttt cgt      816
Trp Ala Met Tyr Lys Val Lys Gly Thr Asn Glu Glu Met Val Phe Arg
            260                 265                 270

gga aat gtt gat aac aac aca cca tat gct aat tct ttc aca ccc cca      864
Gly Asn Val Asp Asn Asn Thr Pro Tyr Ala Asn Ser Phe Thr Pro Pro
        275                 280                 285

atc aaa gct cag tat gta aga ctc tac ccc caa att tgt cga agg cat      912
Ile Lys Ala Gln Tyr Val Arg Leu Tyr Pro Gln Ile Cys Arg Arg His
    290                 295                 300

tgt act tta aga atg gaa ctt ctt ggc tgt gag ctc tca ggc tgt tca      960
Cys Thr Leu Arg Met Glu Leu Leu Gly Cys Glu Leu Ser Gly Cys Ser
305                 310                 315                 320

gaa cct ttg ggg atg aaa tca ggg cat ata caa gac tac cag atc act     1008
Glu Pro Leu Gly Met Lys Ser Gly His Ile Gln Asp Tyr Gln Ile Thr
                325                 330                 335

gcc tcc agc gtc ttc aga aca ctc aac atg gac atg                     1044
Ala Ser Ser Val Phe Arg Thr Leu Asn Met Asp Met
            340                 345

<210>  9
<211>  348
<212>  PRT
<213>  Mus musculus

<400>  9

Met Lys His Leu Val Ala Ala Trp Leu Leu Val Gly Leu Ser Leu Gly

1               5                   10                  15

Val Pro Gln Phe Gly Lys Gly Asp Ile Cys Asn Pro Asn Pro Cys Glu
            20                  25                  30

Asn Gly Gly Ile Cys Leu Ser Gly Leu Ala Asp Asp Ser Phe Ser Cys
        35                  40                  45

Glu Cys Pro Glu Gly Phe Ala Gly Pro Asn Cys Ser Ser Val Val Glu
    50                  55                  60

Val Ala Ser Asp Glu Glu Lys Pro Thr Ser Ala Gly Pro Cys Ile Pro
65                  70                  75                  80

Asn Pro Cys His Asn Gly Gly Thr Cys Glu Ile Ser Glu Ala Tyr Arg
                85                  90                  95

Gly Asp Thr Phe Ile Gly Tyr Val Cys Lys Cys Pro Arg Gly Phe Asn
            100                 105                 110

Gly Ile His Cys Gln His Asn Ile Asn Glu Cys Glu Ala Glu Pro Cys
        115                 120                 125

Arg Asn Gly Gly Ile Cys Thr Asp Leu Val Ala Asn Tyr Ser Cys Glu
    130                 135                 140

Cys Pro Gly Glu Phe Met Gly Arg Asn Cys Gln Tyr Lys Cys Ser Gly
145                 150                 155                 160

Pro Leu Gly Ile Glu Gly Gly Ile Ile Ser Asn Gln Gln Ile Thr Ala
                165                 170                 175

Ser Ser Thr His Arg Ala Leu Phe Gly Leu Arg Lys Trp Tyr Pro Tyr
            180                 185                 190

Tyr Ala Arg Leu Asn Lys Lys Gly Leu Ile Asn Ala Trp Thr Ala Ala
        195                 200                 205

Glu Asn Asp Arg Trp Pro Trp Ile Gln Ile Asn Leu Gln Arg Lys Met
    210                 215                 220

Arg Val Thr Gly Val Ile Thr Gln Gly Ala Lys Arg Ile Gly Ser Pro
225                 230                 235                 240

Glu Tyr Ile Lys Ser Tyr Lys Ile Ala Tyr Ser Asn Asp Gly Lys Thr
                245                 250                 255

Trp Ala Met Tyr Lys Val Lys Gly Thr Asn Glu Glu Met Val Phe Arg
            260                 265                 270

Gly Asn Val Asp Asn Asn Thr Pro Tyr Ala Asn Ser Phe Thr Pro Pro
        275                 280                 285

Ile Lys Ala Gln Tyr Val Arg Leu Tyr Pro Gln Ile Cys Arg Arg His
    290                 295                 300

Cys Thr Leu Arg Met Glu Leu Leu Gly Cys Glu Leu Ser Gly Cys Ser
305                 310                 315                 320

Glu Pro Leu Gly Met Lys Ser Gly His Ile Gln Asp Tyr Gln Ile Thr
                325                 330                 335

Ala Ser Ser Val Phe Arg Thr Leu Asn Met Asp Met
            340                 345

<210>  10
<211>  1095
<212>  DNA
<213>  Mus musculus

<220>
<221>  CDS
<222>  (1)..(1095)

<400>  10
atg aag cac ttg gta gca gcc tgg ctt ttg gtt gga ctc agc ctc ggg       48
Met Lys His Leu Val Ala Ala Trp Leu Leu Val Gly Leu Ser Leu Gly
1               5                   10                  15

gtg ccc cag ttc ggc aaa ggt gac att tgc aac ccg aac ccc tgt gaa       96
Val Pro Gln Phe Gly Lys Gly Asp Ile Cys Asn Pro Asn Pro Cys Glu
            20                  25                  30

aat ggt ggc atc tgt ctg tca gga ctg gct gat gat tcc ttt tcc tgt      144
Asn Gly Gly Ile Cys Leu Ser Gly Leu Ala Asp Asp Ser Phe Ser Cys
        35                  40                  45

gag tgt cca gaa ggc ttc gca ggt ccg aac tgc tct agt gtt gtg gag      192
Glu Cys Pro Glu Gly Phe Ala Gly Pro Asn Cys Ser Ser Val Val Glu
    50                  55                  60

gtt gca tca gat gaa gaa aag cct act tca gca ggt ccc tgc atc cct      240
Val Ala Ser Asp Glu Glu Lys Pro Thr Ser Ala Gly Pro Cys Ile Pro
65                  70                  75                  80

aac cca tgc cat aac gga gga acc tgt gag ata agc gaa gcc tat cga      288
Asn Pro Cys His Asn Gly Gly Thr Cys Glu Ile Ser Glu Ala Tyr Arg
                85                  90                  95

gga gac aca ttc ata ggc tat gtt tgt aaa tgt cct cgg gga ttt aat      336
Gly Asp Thr Phe Ile Gly Tyr Val Cys Lys Cys Pro Arg Gly Phe Asn
            100                 105                 110

ggg att cac tgt cag cac aat ata aat gaa tgt gaa gct gag cct tgc      384
Gly Ile His Cys Gln His Asn Ile Asn Glu Cys Glu Ala Glu Pro Cys
        115                 120                 125

aga aat ggc gga ata tgt acc gac ctt gtt gct aac tac tct tgt gaa     432
Arg Asn Gly Gly Ile Cys Thr Asp Leu Val Ala Asn Tyr Ser Cys Glu
    130                 135                 140

tgc cca gga gaa ttt atg gga cga aat tgt caa tat aaa tgc tct ggg     480
Cys Pro Gly Glu Phe Met Gly Arg Asn Cys Gln Tyr Lys Cys Ser Gly
145                 150                 155                 160

cca ttg gga atc gaa ggt ggg atc ata tct aat cag caa atc aca gct     528
Pro Leu Gly Ile Glu Gly Gly Ile Ile Ser Asn Gln Gln Ile Thr Ala
                165                 170                 175

tca tct act cac cga gct ctt ttt gga ctc cgg aag tgg tat ccc tac     576
Ser Ser Thr His Arg Ala Leu Phe Gly Leu Arg Lys Trp Tyr Pro Tyr
            180                 185                 190

tat gct cga ctt aat aag aag ggc ctt ata aat gcc tgg aca gct gct     624
Tyr Ala Arg Leu Asn Lys Lys Gly Leu Ile Asn Ala Trp Thr Ala Ala
        195                 200                 205

gaa aat gac aga tgg cca tgg att cag ata aat ttg caa aga aaa atg     672
Glu Asn Asp Arg Trp Pro Trp Ile Gln Ile Asn Leu Gln Arg Lys Met
    210                 215                 220

aga gtc act ggt gtt att acc caa gga gca aaa agg att gga agc cca     720
Arg Val Thr Gly Val Ile Thr Gln Gly Ala Lys Arg Ile Gly Ser Pro
225                 230                 235                 240

gag tac ata aaa tcc tac aaa att gcc tac agc aat gac ggg aag acc     768
Glu Tyr Ile Lys Ser Tyr Lys Ile Ala Tyr Ser Asn Asp Gly Lys Thr
                245                 250                 255

tgg gca atg tac aaa gta aaa ggc acc aat gaa gag atg gtc ttt cgt     816
Trp Ala Met Tyr Lys Val Lys Gly Thr Asn Glu Glu Met Val Phe Arg
            260                 265                 270

gga aat gtt gat aac aac aca cca tat gct aat tct ttc aca ccc cca     864
Gly Asn Val Asp Asn Asn Thr Pro Tyr Ala Asn Ser Phe Thr Pro Pro
        275                 280                 285

atc aaa gct cag tat gta aga ctc tac ccc caa att tgt cga agg cat     912
Ile Lys Ala Gln Tyr Val Arg Leu Tyr Pro Gln Ile Cys Arg Arg His
    290                 295                 300

tgt act tta aga atg gaa ctt ctt ggc tgt gag ctc tca ggc tgt tca     960
Cys Thr Leu Arg Met Glu Leu Leu Gly Cys Glu Leu Ser Gly Cys Ser
305                 310                 315                 320

gaa cct ttg ggg atg aaa tca ggg cat ata caa gac tac cag atc act    1008
Glu Pro Leu Gly Met Lys Ser Gly His Ile Gln Asp Tyr Gln Ile Thr
                325                 330                 335

gcc tcc agc gtc ttc aga aca ctc aac atg gac atg ttt act tgg gaa    1056
Ala Ser Ser Val Phe Arg Thr Leu Asn Met Asp Met Phe Thr Trp Glu
            340                 345                 350

cca agg aaa gcc agg ctg gac aag caa ggc aaa gta aat                1095
Pro Arg Lys Ala Arg Leu Asp Lys Gln Gly Lys Val Asn
        355                 360                 365


<210>  11
<211>  365
<212>  PRT
<213>  Mus musculus

<400>  11

Met Lys His Leu Val Ala Ala Trp Leu Leu Val Gly Leu Ser Leu Gly
1               5                   10                  15

Val Pro Gln Phe Gly Lys Gly Asp Ile Cys Asn Pro Asn Pro Cys Glu
            20                  25                  30

Asn Gly Gly Ile Cys Leu Ser Gly Leu Ala Asp Asp Ser Phe Ser Cys
        35                  40                  45

Glu Cys Pro Glu Gly Phe Ala Gly Pro Asn Cys Ser Ser Val Val Glu
    50                  55                  60

Val Ala Ser Asp Glu Glu Lys Pro Thr Ser Ala Gly Pro Cys Ile Pro
65                  70                  75                  80

Asn Pro Cys His Asn Gly Gly Thr Cys Glu Ile Ser Glu Ala Tyr Arg
                85                  90                  95

Gly Asp Thr Phe Ile Gly Tyr Val Cys Lys Cys Pro Arg Gly Phe Asn
            100                 105                 110

Gly Ile His Cys Gln His Asn Ile Asn Glu Cys Glu Ala Glu Pro Cys
        115                 120                 125

Arg Asn Gly Gly Ile Cys Thr Asp Leu Val Ala Asn Tyr Ser Cys Glu
    130                 135                 140

Cys Pro Gly Glu Phe Met Gly Arg Asn Cys Gln Tyr Lys Cys Ser Gly
145                 150                 155                 160

Pro Leu Gly Ile Glu Gly Gly Ile Ile Ser Asn Gln Gln Ile Thr Ala
                165                 170                 175

Ser Ser Thr His Arg Ala Leu Phe Gly Leu Arg Lys Trp Tyr Pro Tyr
            180                 185                 190

Tyr Ala Arg Leu Asn Lys Lys Gly Leu Ile Asn Ala Trp Thr Ala Ala
        195                 200                 205

Glu Asn Asp Arg Trp Pro Trp Ile Gln Ile Asn Leu Gln Arg Lys Met
    210                 215                 220

Arg Val Thr Gly Val Ile Thr Gln Gly Ala Lys Arg Ile Gly Ser Pro
225                 230                 235                 240

Glu Tyr Ile Lys Ser Tyr Lys Ile Ala Tyr Ser Asn Asp Gly Lys Thr
                245                 250                 255

Trp Ala Met Tyr Lys Val Lys Gly Thr Asn Glu Glu Met Val Phe Arg
            260                 265                 270

Gly Asn Val Asp Asn Asn Thr Pro Tyr Ala Asn Ser Phe Thr Pro Pro
        275                 280                 285

Ile Lys Ala Gln Tyr Val Arg Leu Tyr Pro Gln Ile Cys Arg Arg His
    290                 295                 300

Cys Thr Leu Arg Met Glu Leu Leu Gly Cys Glu Leu Ser Gly Cys Ser
305                 310                 315                 320

Glu Pro Leu Gly Met Lys Ser Gly His Ile Gln Asp Tyr Gln Ile Thr
                325                 330                 335

Ala Ser Ser Val Phe Arg Thr Leu Asn Met Asp Met Phe Thr Trp Glu
            340                 345                 350

Pro Arg Lys Ala Arg Leu Asp Lys Gln Gly Lys Val Asn
        355                 360                 365

<210> 12
<211> 1104
<212> DNA
<213> Mus musculus

<220>
<221> CDS
<222> (1)..(1104)

<400> 12
atg aag cac ttg gta gca gcc tgg ctt ttg gtt gga ctc agc ctc ggg 48
Met Lys His Leu Val Ala Ala Trp Leu Leu Val Gly Leu Ser Leu Gly
1               5                   10                  15

gtg ccc cag ttc ggc aaa ggt gac att tgc aac ccg aac ccc tgt gaa 96
Val Pro Gln Phe Gly Lys Gly Asp Ile Cys Asn Pro Asn Pro Cys Glu
            20                  25                  30

aat ggt ggc atc tgt ctg tca gga ctg gct gat gat tcc ttt tcc tgt 144
Asn Gly Gly Ile Cys Leu Ser Gly Leu Ala Asp Asp Ser Phe Ser Cys
        35                  40                  45

gag tgt cca gaa ggc ttc gca ggt ccg aac tgc tct agt gtt gtg gag 192
Glu Cys Pro Glu Gly Phe Ala Gly Pro Asn Cys Ser Ser Val Val Glu
    50                  55                  60

gtt gca tca gat gaa gaa aag cct act tca gca ggt ccc tgc atc cct 240
Val Ala Ser Asp Glu Glu Lys Pro Thr Ser Ala Gly Pro Cys Ile Pro
65                  70                  75                  80

aac cca tgc cat aac gga gga acc tgt gag ata agc gaa gcc tat cga 288
Asn Pro Cys His Asn Gly Gly Thr Cys Glu Ile Ser Glu Ala Tyr Arg
                85                  90                  95

gga gac aca ttc ata ggc tat gtt tgt aaa tgt cct cgg gga ttt aat 336
Gly Asp Thr Phe Ile Gly Tyr Val Cys Lys Cys Pro Arg Gly Phe Asn
            100                 105                 110

ggg att cac tgt cag cac aat ata aat gaa tgt gaa gct gag cct tgc 384
Gly Ile His Cys Gln His Asn Ile Asn Glu Cys Glu Ala Glu Pro Cys
        115                 120                 125

aga aat ggc gga ata tgt acc gac ctt gtt gct aac tac tct tgt gaa 432
Arg Asn Gly Gly Ile Cys Thr Asp Leu Val Ala Asn Tyr Ser Cys Glu
    130                 135                 140

tgc cca gga gaa ttt atg gga cga aat tgt caa tat aaa tgc tct ggg 480
Cys Pro Gly Glu Phe Met Gly Arg Asn Cys Gln Tyr Lys Cys Ser Gly
145                 150                 155                 160

cca ttg gga atc gaa ggt ggg atc ata tct aat cag caa atc aca gct 528
Pro Leu Gly Ile Glu Gly Gly Ile Ile Ser Asn Gln Gln Ile Thr Ala
                165                 170                 175

tca tct act cac cga gct ctt ttt gga ctc cgg aag tgg tat ccc tac 576
Ser Ser Thr His Arg Ala Leu Phe Gly Leu Arg Lys Trp Tyr Pro Tyr
            180                 185                 190

tat gct cga ctt aat aag aag ggc ctt ata aat gcc tgg aca gct gct 624
Tyr Ala Arg Leu Asn Lys Lys Gly Leu Ile Asn Ala Trp Thr Ala Ala
        195                 200                 205

gaa aat gac aga tgg cca tgg att cag ata aat ttg caa aga aaa atg 672
Glu Asn Asp Arg Trp Pro Trp Ile Gln Ile Asn Leu Gln Arg Lys Met
    210                 215                 220

aga gtc act ggt gtt att acc caa gga gca aaa agg att gga agc cca 720
Arg Val Thr Gly Val Ile Thr Gln Gly Ala Lys Arg Ile Gly Ser Pro
225                 230                 235                 240

gag tac ata aaa tcc tac aaa att gcc tac agc aat gac ggg aag acc 768
Glu Tyr Ile Lys Ser Tyr Lys Ile Ala Tyr Ser Asn Asp Gly Lys Thr
                245                 250                 255

tgg gca atg tac aaa gta aaa ggc acc aat gaa gag atg gtc ttt cgt 816
Trp Ala Met Tyr Lys Val Lys Gly Thr Asn Glu Glu Met Val Phe Arg
            260                 265                 270

gga aat gtt gat aac aac aca cca tat gct aat tct ttc aca ccc cca 864
Gly Asn Val Asp Asn Asn Thr Pro Tyr Ala Asn Ser Phe Thr Pro Pro
        275                 280                 285

atc aaa gct cag tat gta aga ctc tac ccc caa att tgt cga agg cat 912
Ile Lys Ala Gln Tyr Val Arg Leu Tyr Pro Gln Ile Cys Arg Arg His
    290                 295                 300

tgt act tta aga atg gaa ctt ctt ggc tgt gag ctc tca ggc tgt tca 960
Cys Thr Leu Arg Met Glu Leu Leu Gly Cys Glu Leu Ser Gly Cys Ser
305                 310                 315                 320

gaa cct ttg ggg atg aaa tca ggg cat ata caa gac tac cag atc act 1008
Glu Pro Leu Gly Met Lys Ser Gly His Ile Gln Asp Tyr Gln Ile Thr
                325                 330                 335

gcc tcc agc gtc ttc aga aca ctc aac atg gac atg ttt act tgg gaa 1056
Ala Ser Ser Val Phe Arg Thr Leu Asn Met Asp Met Phe Thr Trp Glu
            340                 345                 350

cca agg aaa gcc agg ctg gac aag caa ggc aaa gta aat gcc tgg act 1104

Pro Arg Lys Ala Arg Leu Asp Lys Gln Gly Lys Val Asn Ala Trp Thr
        355                 360                 365

<210>  13
<211>  368
<212>  PRT
<213>  Mus musculus

<400>  13

Met Lys His Leu Val Ala Ala Trp Leu Leu Val Gly Leu Ser Leu Gly
1               5                   10                  15

Val Pro Gln Phe Gly Lys Gly Asp Ile Cys Asn Pro Asn Pro Cys Glu
            20                  25                  30

Asn Gly Gly Ile Cys Leu Ser Gly Leu Ala Asp Asp Ser Phe Ser Cys
        35                  40                  45

Glu Cys Pro Glu Gly Phe Ala Gly Pro Asn Cys Ser Ser Val Val Glu
    50                  55                  60

Val Ala Ser Asp Glu Glu Lys Pro Thr Ser Ala Gly Pro Cys Ile Pro
65                  70                  75                  80

Asn Pro Cys His Asn Gly Gly Thr Cys Glu Ile Ser Glu Ala Tyr Arg
                85                  90                  95

Gly Asp Thr Phe Ile Gly Tyr Val Cys Lys Cys Pro Arg Gly Phe Asn
            100                 105                 110

Gly Ile His Cys Gln His Asn Ile Asn Glu Cys Glu Ala Glu Pro Cys
        115                 120                 125

Arg Asn Gly Gly Ile Cys Thr Asp Leu Val Ala Asn Tyr Ser Cys Glu
    130                 135                 140

Cys Pro Gly Glu Phe Met Gly Arg Asn Cys Gln Tyr Lys Cys Ser Gly
145                 150                 155                 160

Pro Leu Gly Ile Glu Gly Gly Ile Ile Ser Asn Gln Gln Ile Thr Ala
                165                 170                 175

Ser Ser Thr His Arg Ala Leu Phe Gly Leu Arg Lys Trp Tyr Pro Tyr
            180                 185                 190

Tyr Ala Arg Leu Asn Lys Lys Gly Leu Ile Asn Ala Trp Thr Ala Ala
        195                 200                 205

Glu Asn Asp Arg Trp Pro Trp Ile Gln Ile Asn Leu Gln Arg Lys Met
    210                 215                 220

Arg Val Thr Gly Val Ile Thr Gln Gly Ala Lys Arg Ile Gly Ser Pro
225                 230                 235                 240

Glu Tyr Ile Lys Ser Tyr Lys Ile Ala Tyr Ser Asn Asp Gly Lys Thr
                245                 250                 255

Trp Ala Met Tyr Lys Val Lys Gly Thr Asn Glu Glu Met Val Phe Arg
            260                 265                 270

Gly Asn Val Asp Asn Asn Thr Pro Tyr Ala Asn Ser Phe Thr Pro Pro
        275                 280                 285

Ile Lys Ala Gln Tyr Val Arg Leu Tyr Pro Gln Ile Cys Arg Arg His
    290                 295                 300

Cys Thr Leu Arg Met Glu Leu Leu Gly Cys Glu Leu Ser Gly Cys Ser
305                 310                 315                 320

Glu Pro Leu Gly Met Lys Ser Gly His Ile Gln Asp Tyr Gln Ile Thr
                325                 330                 335

Ala Ser Ser Val Phe Arg Thr Leu Asn Met Asp Met Phe Thr Trp Glu
            340                 345                 350

Pro Arg Lys Ala Arg Leu Asp Lys Gln Gly Lys Val Asn Ala Trp Thr
        355                 360                 365

<210>  14
<211>  1155
<212>  DNA
<213>  Mus musculus

<220>
<221>  CDS
<222>  (1)..(1155)

<400>  14
atg aag cac ttg gta gca gcc tgg ctt ttg gtt gga ctc agc ctc ggg       48
Met Lys His Leu Val Ala Ala Trp Leu Leu Val Gly Leu Ser Leu Gly
1               5                   10                  15

gtg ccc cag ttc ggc aaa ggt gac att tgc aac ccg aac ccc tgt gaa       96
Val Pro Gln Phe Gly Lys Gly Asp Ile Cys Asn Pro Asn Pro Cys Glu
            20                  25                  30

aat ggt ggc atc tgt ctg tca gga ctg gct gat gat tcc ttt tcc tgt      144
Asn Gly Gly Ile Cys Leu Ser Gly Leu Ala Asp Asp Ser Phe Ser Cys
        35                  40                  45

gag tgt cca gaa ggc ttc gca ggt ccg aac tgc tct agt gtt gtg gag      192
Glu Cys Pro Glu Gly Phe Ala Gly Pro Asn Cys Ser Ser Val Val Glu
    50                  55                  60

gtt gca tca gat gaa gaa aag cct act tca gca ggt ccc tgc atc cct     240
Val Ala Ser Asp Glu Glu Lys Pro Thr Ser Ala Gly Pro Cys Ile Pro
65                  70                  75                  80

aac cca tgc cat aac gga gga acc tgt gag ata agc gaa gcc tat cga     288
Asn Pro Cys His Asn Gly Gly Thr Cys Glu Ile Ser Glu Ala Tyr Arg
                85                  90                  95

gga gac aca ttc ata ggc tat gtt tgt aaa tgt cct cgg gga ttt aat     336
Gly Asp Thr Phe Ile Gly Tyr Val Cys Lys Cys Pro Arg Gly Phe Asn
            100                 105                 110

ggg att cac tgt cag cac aat ata aat gaa tgt gaa gct gag cct tgc     384
Gly Ile His Cys Gln His Asn Ile Asn Glu Cys Glu Ala Glu Pro Cys
        115                 120                 125

aga aat ggc gga ata tgt acc gac ctt gtt gct aac tac tct tgt gaa     432
Arg Asn Gly Gly Ile Cys Thr Asp Leu Val Ala Asn Tyr Ser Cys Glu
    130                 135                 140

tgc cca gga gaa ttt atg gga cga aat tgt caa tat aaa tgc tct ggg     480
Cys Pro Gly Glu Phe Met Gly Arg Asn Cys Gln Tyr Lys Cys Ser Gly
145                 150                 155                 160

cca ttg gga atc gaa ggt ggg atc ata tct aat cag caa atc aca gct     528
Pro Leu Gly Ile Glu Gly Gly Ile Ile Ser Asn Gln Gln Ile Thr Ala
                165                 170                 175

tca tct act cac cga gct ctt ttt gga ctc cgg aag tgg tat ccc tac     576
Ser Ser Thr His Arg Ala Leu Phe Gly Leu Arg Lys Trp Tyr Pro Tyr
            180                 185                 190

tat gct cga ctt aat aag aag ggc ctt ata aat gcc tgg aca gct gct     624
Tyr Ala Arg Leu Asn Lys Lys Gly Leu Ile Asn Ala Trp Thr Ala Ala
        195                 200                 205

gaa aat gac aga tgg cca tgg att cag ata aat ttg caa aga aaa atg     672
Glu Asn Asp Arg Trp Pro Trp Ile Gln Ile Asn Leu Gln Arg Lys Met
    210                 215                 220

aga gtc act ggt gtt att acc caa gga gca aaa agg att gga agc cca     720
Arg Val Thr Gly Val Ile Thr Gln Gly Ala Lys Arg Ile Gly Ser Pro
225                 230                 235                 240

gag tac ata aaa tcc tac aaa att gcc tac agc aat gac ggg aag acc     768
Glu Tyr Ile Lys Ser Tyr Lys Ile Ala Tyr Ser Asn Asp Gly Lys Thr
                245                 250                 255

tgg gca atg tac aaa gta aaa ggc acc aat gaa gag atg gtc ttt cgt     816
Trp Ala Met Tyr Lys Val Lys Gly Thr Asn Glu Glu Met Val Phe Arg
            260                 265                 270

gga aat gtt gat aac aac aca cca tat gct aat tct ttc aca ccc cca     864
Gly Asn Val Asp Asn Asn Thr Pro Tyr Ala Asn Ser Phe Thr Pro Pro
        275                 280                 285

atc aaa gct cag tat gta aga ctc tac ccc caa att tgt cga agg cat     912
Ile Lys Ala Gln Tyr Val Arg Leu Tyr Pro Gln Ile Cys Arg Arg His
    290                 295                 300

tgt act tta aga atg gaa ctt ctt ggc tgt gag ctc tca ggc tgt tca     960
Cys Thr Leu Arg Met Glu Leu Leu Gly Cys Glu Leu Ser Gly Cys Ser
305                 310                 315                 320

gaa cct ttg ggg atg aaa tca ggg cat ata caa gac tac cag atc act    1008

Glu Pro Leu Gly Met Lys Ser Gly His Ile Gln Asp Tyr Gln Ile Thr
                325                 330                 335

gcc tcc agc gtc ttc aga aca ctc aac atg gac atg ttt act tgg gaa 1056
Ala Ser Ser Val Phe Arg Thr Leu Asn Met Asp Met Phe Thr Trp Glu
            340                 345                 350

cca agg aaa gcc agg ctg gac aag caa ggc aaa gta aat gcc tgg act 1104
Pro Arg Lys Ala Arg Leu Asp Lys Gln Gly Lys Val Asn Ala Trp Thr
        355                 360                 365

tcc ggc cat aac gac cag tca caa tgg tta cag gtt gat ctt ctt gtc 1152
Ser Gly His Asn Asp Gln Ser Gln Trp Leu Gln Val Asp Leu Leu Val
    370                 375                 380

cct 1155
Pro
385

<210> 15
<211> 385
<212> PRT
<213> Mus musculus

<400> 15

Met Lys His Leu Val Ala Ala Trp Leu Leu Val Gly Leu Ser Leu Gly
1               5                   10                  15

Val Pro Gln Phe Gly Lys Gly Asp Ile Cys Asn Pro Asn Pro Cys Glu
            20                  25                  30

Asn Gly Gly Ile Cys Leu Ser Gly Leu Ala Asp Asp Ser Phe Ser Cys
        35                  40                  45

Glu Cys Pro Glu Gly Phe Ala Gly Pro Asn Cys Ser Ser Val Val Glu
    50                  55                  60

Val Ala Ser Asp Glu Glu Lys Pro Thr Ser Ala Gly Pro Cys Ile Pro
65                  70                  75                  80

Asn Pro Cys His Asn Gly Gly Thr Cys Glu Ile Ser Glu Ala Tyr Arg
                85                  90                  95

Gly Asp Thr Phe Ile Gly Tyr Val Cys Lys Cys Pro Arg Gly Phe Asn
            100                 105                 110

Gly Ile His Cys Gln His Asn Ile Asn Glu Cys Glu Ala Glu Pro Cys
        115                 120                 125

Arg Asn Gly Gly Ile Cys Thr Asp Leu Val Ala Asn Tyr Ser Cys Glu
    130                 135                 140

Cys Pro Gly Glu Phe Met Gly Arg Asn Cys Gln Tyr Lys Cys Ser Gly
145                 150                 155                 160

Pro Leu Gly Ile Glu Gly Gly Ile Ile Ser Asn Gln Gln Ile Thr Ala
                165                 170                 175

Ser Ser Thr His Arg Ala Leu Phe Gly Leu Arg Lys Trp Tyr Pro Tyr
            180                 185                 190

Tyr Ala Arg Leu Asn Lys Lys Gly Leu Ile Asn Ala Trp Thr Ala Ala
        195                 200                 205

Glu Asn Asp Arg Trp Pro Trp Ile Gln Ile Asn Leu Gln Arg Lys Met
    210                 215                 220

Arg Val Thr Gly Val Ile Thr Gln Gly Ala Lys Arg Ile Gly Ser Pro
225                 230                 235                 240

Glu Tyr Ile Lys Ser Tyr Lys Ile Ala Tyr Ser Asn Asp Gly Lys Thr
                245                 250                 255

Trp Ala Met Tyr Lys Val Lys Gly Thr Asn Glu Glu Met Val Phe Arg
            260                 265                 270

Gly Asn Val Asp Asn Asn Thr Pro Tyr Ala Asn Ser Phe Thr Pro Pro
        275                 280                 285

Ile Lys Ala Gln Tyr Val Arg Leu Tyr Pro Gln Ile Cys Arg Arg His
    290                 295                 300

Cys Thr Leu Arg Met Glu Leu Leu Gly Cys Glu Leu Ser Gly Cys Ser
305                 310                 315                 320

Glu Pro Leu Gly Met Lys Ser Gly His Ile Gln Asp Tyr Gln Ile Thr
                325                 330                 335

Ala Ser Ser Val Phe Arg Thr Leu Asn Met Asp Met Phe Thr Trp Glu
            340                 345                 350

Pro Arg Lys Ala Arg Leu Asp Lys Gln Gly Lys Val Asn Ala Trp Thr
        355                 360                 365

Ser Gly His Asn Asp Gln Ser Gln Trp Leu Gln Val Asp Leu Leu Val
    370                 375                 380

Pro
385

<210>  16
<211>  678
<212>  DNA

<213>  Mus musculus

<220>
<221>  CDS
<222>  (1)..(678)

<400>  16
tgt gaa gct gag cct tgc aga aat ggc gga ata tgt acc gac ctt gtt      48
Cys Glu Ala Glu Pro Cys Arg Asn Gly Gly Ile Cys Thr Asp Leu Val
1               5                   10                  15

gct aac tac tct tgt gaa tgc cca gga gaa ttt atg gga cga aat tgt      96
Ala Asn Tyr Ser Cys Glu Cys Pro Gly Glu Phe Met Gly Arg Asn Cys
            20                  25                  30

caa tat aaa tgc tct ggg cca ttg gga atc gaa ggt ggg atc ata tct      144
Gln Tyr Lys Cys Ser Gly Pro Leu Gly Ile Glu Gly Gly Ile Ile Ser
        35                  40                  45

aat cag caa atc aca gct tca tct act cac cga gct ctt ttt gga ctc      192
Asn Gln Gln Ile Thr Ala Ser Ser Thr His Arg Ala Leu Phe Gly Leu
    50                  55                  60

cgg aag tgg tat ccc tac tat gct cga ctt aat aag aag ggc ctt ata      240
Arg Lys Trp Tyr Pro Tyr Tyr Ala Arg Leu Asn Lys Lys Gly Leu Ile
65                  70                  75                  80

aat gcc tgg aca gct gct gaa aat gac aga tgg cca tgg att cag ata      288
Asn Ala Trp Thr Ala Ala Glu Asn Asp Arg Trp Pro Trp Ile Gln Ile
                85                  90                  95

aat ttg caa aga aaa atg aga gtc act ggt gtt att acc caa gga gca      336
Asn Leu Gln Arg Lys Met Arg Val Thr Gly Val Ile Thr Gln Gly Ala
            100                 105                 110

aaa agg att gga agc cca gag tac ata aaa tcc tac aaa att gcc tac      384
Lys Arg Ile Gly Ser Pro Glu Tyr Ile Lys Ser Tyr Lys Ile Ala Tyr
        115                 120                 125

agc aat gac ggg aag acc tgg gca atg tac aaa gta aaa ggc acc aat      432
Ser Asn Asp Gly Lys Thr Trp Ala Met Tyr Lys Val Lys Gly Thr Asn
    130                 135                 140

gaa gag atg gtc ttt cgt gga aat gtt gat aac aac aca cca tat gct      480
Glu Glu Met Val Phe Arg Gly Asn Val Asp Asn Asn Thr Pro Tyr Ala
145                 150                 155                 160

aat tct ttc aca ccc cca atc aaa gct cag tat gta aga ctc tac ccc      528
Asn Ser Phe Thr Pro Pro Ile Lys Ala Gln Tyr Val Arg Leu Tyr Pro
                165                 170                 175

caa att tgt cga agg cat tgt act tta aga atg gaa ctt ctt ggc tgt      576
Gln Ile Cys Arg Arg His Cys Thr Leu Arg Met Glu Leu Leu Gly Cys
            180                 185                 190

gag ctc tca ggc tgt tca gaa cct ttg ggg atg aaa tca ggg cat ata      624
Glu Leu Ser Gly Cys Ser Glu Pro Leu Gly Met Lys Ser Gly His Ile
        195                 200                 205

caa gac tac cag atc act gcc tcc agc gtc ttc aga aca ctc aac atg      672
Gln Asp Tyr Gln Ile Thr Ala Ser Ser Val Phe Arg Thr Leu Asn Met
    210                 215                 220

gac atg                                                              678

Asp Met
225

<210> 17
<211> 226
<212> PRT
<213> Mus musculus

<400> 17

Cys Glu Ala Glu Pro Cys Arg Asn Gly Gly Ile Cys Thr Asp Leu Val
1               5                   10                  15

Ala Asn Tyr Ser Cys Glu Cys Pro Gly Glu Phe Met Gly Arg Asn Cys
            20                  25                  30

Gln Tyr Lys Cys Ser Gly Pro Leu Gly Ile Glu Gly Gly Ile Ile Ser
        35                  40                  45

Asn Gln Gln Ile Thr Ala Ser Ser Thr His Arg Ala Leu Phe Gly Leu
    50                  55                  60

Arg Lys Trp Tyr Pro Tyr Tyr Ala Arg Leu Asn Lys Lys Gly Leu Ile
65                  70                  75                  80

Asn Ala Trp Thr Ala Ala Glu Asn Asp Arg Trp Pro Trp Ile Gln Ile
                85                  90                  95

Asn Leu Gln Arg Lys Met Arg Val Thr Gly Val Ile Thr Gln Gly Ala
            100                 105                 110

Lys Arg Ile Gly Ser Pro Glu Tyr Ile Lys Ser Tyr Lys Ile Ala Tyr
        115                 120                 125

Ser Asn Asp Gly Lys Thr Trp Ala Met Tyr Lys Val Lys Gly Thr Asn
    130                 135                 140

Glu Glu Met Val Phe Arg Gly Asn Val Asp Asn Asn Thr Pro Tyr Ala
145                 150                 155                 160

Asn Ser Phe Thr Pro Pro Ile Lys Ala Gln Tyr Val Arg Leu Tyr Pro
                165                 170                 175

Gln Ile Cys Arg Arg His Cys Thr Leu Arg Met Glu Leu Leu Gly Cys
            180                 185                 190

Glu Leu Ser Gly Cys Ser Glu Pro Leu Gly Met Lys Ser Gly His Ile
        195                 200                 205

Gln Asp Tyr Gln Ile Thr Ala Ser Ser Val Phe Arg Thr Leu Asn Met
    210                 215                 220

Asp Met
225

<210> 18
<211> 285
<212> DNA
<213> Mus musculus

<220>
<221> CDS
<222> (1)..(285)

<400> 18
tgt gaa gct gag cct tgc aga aat ggc gga ata tgt acc gac ctt gtt 48
Cys Glu Ala Glu Pro Cys Arg Asn Gly Gly Ile Cys Thr Asp Leu Val
1               5                   10                  15

gct aac tac tct tgt gaa tgc cca gga gaa ttt atg gga cga aat tgt 96
Ala Asn Tyr Ser Cys Glu Cys Pro Gly Glu Phe Met Gly Arg Asn Cys
            20                  25                  30

caa tat aaa tgc tct ggg cca ttg gga atc gaa ggt ggg atc ata tct 144
Gln Tyr Lys Cys Ser Gly Pro Leu Gly Ile Glu Gly Gly Ile Ile Ser
        35                  40                  45

aat cag caa atc aca gct tca tct act cac cga gct ctt ttt gga ctc 192
Asn Gln Gln Ile Thr Ala Ser Ser Thr His Arg Ala Leu Phe Gly Leu
    50                  55                  60

cgg aag tgg tat ccc tac tat gct cga ctt aat aag aag ggc ctt ata 240
Arg Lys Trp Tyr Pro Tyr Tyr Ala Arg Leu Asn Lys Lys Gly Leu Ile
65                  70                  75                  80

aat gcc tgg aca gct gct gaa aat gac aga tgg cca tgg att cag 285
Asn Ala Trp Thr Ala Ala Glu Asn Asp Arg Trp Pro Trp Ile Gln
                85                  90                  95

<210> 19
<211> 95
<212> PRT
<213> Mus musculus

<400> 19

Cys Glu Ala Glu Pro Cys Arg Asn Gly Gly Ile Cys Thr Asp Leu Val
1               5                   10                  15

Ala Asn Tyr Ser Cys Glu Cys Pro Gly Glu Phe Met Gly Arg Asn Cys
            20                  25                  30

Gln Tyr Lys Cys Ser Gly Pro Leu Gly Ile Glu Gly Gly Ile Ile Ser
        35                  40                  45

Asn Gln Gln Ile Thr Ala Ser Ser Thr His Arg Ala Leu Phe Gly Leu
    50                  55                  60

Arg Lys Trp Tyr Pro Tyr Tyr Ala Arg Leu Asn Lys Lys Gly Leu Ile
65                  70                  75                  80

Asn Ala Trp Thr Ala Ala Glu Asn Asp Arg Trp Pro Trp Ile Gln
                85                  90                  95

<210>  20
<211>  678
<212>  DNA
<213>  Homo sapiens

<220>
<221>  CDS
<222>  (1)..(678)

<400>  20
tgc gaa gtt gag cct tgc aaa aat ggt gga ata tgt aca gat ctt gtt      48
Cys Glu Val Glu Pro Cys Lys Asn Gly Gly Ile Cys Thr Asp Leu Val
1               5                   10                  15

gct aac tat tcc tgt gag tgc cca ggc gaa ttt atg gga aga aat tgt      96
Ala Asn Tyr Ser Cys Glu Cys Pro Gly Glu Phe Met Gly Arg Asn Cys
            20                  25                  30

caa tac aaa tgc tca ggc cca ctg gga att gaa ggt gga att ata tca      144
Gln Tyr Lys Cys Ser Gly Pro Leu Gly Ile Glu Gly Gly Ile Ile Ser
        35                  40                  45

aac cag caa atc aca gct tcc tct act cac cga gct ctt ttt gga ctc      192
Asn Gln Gln Ile Thr Ala Ser Ser Thr His Arg Ala Leu Phe Gly Leu
    50                  55                  60

caa aaa tgg tat ccc tac tat gca cgt ctt aat aag aag ggg ctt ata      240
Gln Lys Trp Tyr Pro Tyr Tyr Ala Arg Leu Asn Lys Lys Gly Leu Ile
65                  70                  75                  80

aat gcg tgg aca gct gca gaa aat gac aga tgg ccg tgg att cag ata      288
Asn Ala Trp Thr Ala Ala Glu Asn Asp Arg Trp Pro Trp Ile Gln Ile
                85                  90                  95

aat ttg caa agg aaa atg aga gtt act ggt gtg att acc caa gga gcc      336
Asn Leu Gln Arg Lys Met Arg Val Thr Gly Val Ile Thr Gln Gly Ala
            100                 105                 110

aag agg att gga agc cca gag tat ata aaa tcc tac aaa att gcc tac      384
Lys Arg Ile Gly Ser Pro Glu Tyr Ile Lys Ser Tyr Lys Ile Ala Tyr
        115                 120                 125

agt aat gat gga aag act tgg gca atg tac aaa gtg aaa ggc acc aat      432
Ser Asn Asp Gly Lys Thr Trp Ala Met Tyr Lys Val Lys Gly Thr Asn
    130                 135                 140

gaa gac atg gtg ttt cgt gga aac att gat aac aac act cca tat gct      480
Glu Asp Met Val Phe Arg Gly Asn Ile Asp Asn Asn Thr Pro Tyr Ala
145                 150                 155                 160

aac tct ttc aca ccc ccc ata aaa gct cag tat gta aga ctc tat ccc      528
Asn Ser Phe Thr Pro Pro Ile Lys Ala Gln Tyr Val Arg Leu Tyr Pro
                165                 170                 175

caa gtt tgt cga aga cat tgc act ttg cga atg gaa ctt ctt ggc tgt      576
Gln Val Cys Arg Arg His Cys Thr Leu Arg Met Glu Leu Leu Gly Cys

180                 185                 190

gaa ctg tcg ggt tgt tct gag cct ctg ggt atg aaa tca gga cat ata       624
Glu Leu Ser Gly Cys Ser Glu Pro Leu Gly Met Lys Ser Gly His Ile
        195                 200                 205

caa gac tat cag atc act gcc tcc agc atc ttc aga acg ctc aac atg       672
Gln Asp Tyr Gln Ile Thr Ala Ser Ser Ile Phe Arg Thr Leu Asn Met
    210                 215                 220

gac atg                                                               678
Asp Met
225

<210>  21
<211>  226
<212>  PRT
<213>  Homo sapiens

<400>  21

Cys Glu Val Glu Pro Cys Lys Asn Gly Gly Ile Cys Thr Asp Leu Val
1               5                   10                  15

Ala Asn Tyr Ser Cys Glu Cys Pro Gly Glu Phe Met Gly Arg Asn Cys
            20                  25                  30

Gln Tyr Lys Cys Ser Gly Pro Leu Gly Ile Glu Gly Gly Ile Ile Ser
        35                  40                  45

Asn Gln Gln Ile Thr Ala Ser Ser Thr His Arg Ala Leu Phe Gly Leu
    50                  55                  60

Gln Lys Trp Tyr Pro Tyr Tyr Ala Arg Leu Asn Lys Lys Gly Leu Ile
65                  70                  75                  80

Asn Ala Trp Thr Ala Ala Glu Asn Asp Arg Trp Pro Trp Ile Gln Ile
                85                  90                  95

Asn Leu Gln Arg Lys Met Arg Val Thr Gly Val Ile Thr Gln Gly Ala
            100                 105                 110

Lys Arg Ile Gly Ser Pro Glu Tyr Ile Lys Ser Tyr Lys Ile Ala Tyr
        115                 120                 125

Ser Asn Asp Gly Lys Thr Trp Ala Met Tyr Lys Val Lys Gly Thr Asn
    130                 135                 140

Glu Asp Met Val Phe Arg Gly Asn Ile Asp Asn Asn Thr Pro Tyr Ala
145                 150                 155                 160

Asn Ser Phe Thr Pro Pro Ile Lys Ala Gln Tyr Val Arg Leu Tyr Pro
                165                 170                 175

Gln Val Cys Arg Arg His Cys Thr Leu Arg Met Glu Leu Leu Gly Cys
            180                 185                 190

Glu Leu Ser Gly Cys Ser Glu Pro Leu Gly Met Lys Ser Gly His Ile
        195                 200                 205

Gln Asp Tyr Gln Ile Thr Ala Ser Ser Ile Phe Arg Thr Leu Asn Met
    210                 215                 220

Asp Met
225

<210>  22
<211>  1395
<212>  DNA
<213>  Mus musculus

<220>
<221>  CDS
<222>  (1)..(1395)

<400>  22
atg gca gaa tcc ccg gct ctc atc acc atc ttc ctt tta gga tat cta       48
Met Ala Glu Ser Pro Ala Leu Ile Thr Ile Phe Leu Leu Gly Tyr Leu
1               5                   10                  15

ctc agt acc gaa tgt gca gtt ttc ctt gat cgt gaa aat gcc acc aaa       96
Leu Ser Thr Glu Cys Ala Val Phe Leu Asp Arg Glu Asn Ala Thr Lys
            20                  25                  30

att ctt acc cgt cca aag aga tat aat tca gga aaa cta gaa gag ttt      144
Ile Leu Thr Arg Pro Lys Arg Tyr Asn Ser Gly Lys Leu Glu Glu Phe
        35                  40                  45

gtt cga gga aac ctt gaa aga gag tgt ata gaa gaa aga tgt agt ttt      192
Val Arg Gly Asn Leu Glu Arg Glu Cys Ile Glu Glu Arg Cys Ser Phe
    50                  55                  60

gaa gaa gca cga gaa gtt ttt gaa aac act gaa aaa act act gaa ttt      240
Glu Glu Ala Arg Glu Val Phe Glu Asn Thr Glu Lys Thr Thr Glu Phe
65                  70                  75                  80

tgg aag cag tat gtt gat gga gat cag tgt gaa tca aat cct tgt tta      288
Trp Lys Gln Tyr Val Asp Gly Asp Gln Cys Glu Ser Asn Pro Cys Leu
                85                  90                  95

aat ggt gga ata tgc aag gat gat att agt tcc tat gaa tgc tgg tgc      336
Asn Gly Gly Ile Cys Lys Asp Asp Ile Ser Ser Tyr Glu Cys Trp Cys
            100                 105                 110

caa gtt gga ttt gaa gga agg aac tgt gaa tta gat gca acg tgt aac      384
Gln Val Gly Phe Glu Gly Arg Asn Cys Glu Leu Asp Ala Thr Cys Asn
        115                 120                 125

att aaa aat ggc agg tgc aag cag ttt tgt aaa aac agt cct gat aac      432
Ile Lys Asn Gly Arg Cys Lys Gln Phe Cys Lys Asn Ser Pro Asp Asn
    130                 135                 140

aag gta att tgt tcc tgc act gag gga tac caa ctt gca gaa gac cag      480

Lys Val Ile Cys Ser Cys Thr Glu Gly Tyr Gln Leu Ala Glu Asp Gln
145                 150                 155                 160

aag tcc tgt gaa cca aca gtt cca ttt cca tgt ggg aga gct tct att      528
Lys Ser Cys Glu Pro Thr Val Pro Phe Pro Cys Gly Arg Ala Ser Ile
                165                 170                 175

tca tac agt tct aaa aag atc acg aga gct gag act gtt ttc tct aat      576
Ser Tyr Ser Ser Lys Lys Ile Thr Arg Ala Glu Thr Val Phe Ser Asn
            180                 185                 190

atg gac tat gaa aat tct act gaa gct gta ttc att caa gat gac atc      624
Met Asp Tyr Glu Asn Ser Thr Glu Ala Val Phe Ile Gln Asp Asp Ile
        195                 200                 205

act gat ggt gcc att ctt aat aac gtc act gaa agt agt gaa tca ctt      672
Thr Asp Gly Ala Ile Leu Asn Asn Val Thr Glu Ser Ser Glu Ser Leu
    210                 215                 220

aat gac ttc act cga gtt gtt ggt gga gaa aac gca aaa ccg ggt caa      720
Asn Asp Phe Thr Arg Val Val Gly Gly Glu Asn Ala Lys Pro Gly Gln
225                 230                 235                 240

atc cct tgg cag gtc att tta aat ggt gaa att gag gca ttc tgt gga      768
Ile Pro Trp Gln Val Ile Leu Asn Gly Glu Ile Glu Ala Phe Cys Gly
                245                 250                 255

ggt gcc atc att aat gaa aaa tgg att gta act gct gcc cac tgt ctt      816
Gly Ala Ile Ile Asn Glu Lys Trp Ile Val Thr Ala Ala His Cys Leu
            260                 265                 270

aaa cct ggt gat aaa att gag gtt gtt gct ggt gaa tat aac att gat      864
Lys Pro Gly Asp Lys Ile Glu Val Val Ala Gly Glu Tyr Asn Ile Asp
        275                 280                 285

aag aag gaa gac aca gaa caa agg aga aat gtg att cga act atc cct      912
Lys Lys Glu Asp Thr Glu Gln Arg Arg Asn Val Ile Arg Thr Ile Pro
    290                 295                 300

cat cac cag tac aat gca act att aat aag tat agt cat gac att gcc      960
His His Gln Tyr Asn Ala Thr Ile Asn Lys Tyr Ser His Asp Ile Ala
305                 310                 315                 320

ttg ctg gaa ctg gat aaa cct tta ata cta aac agc tat gta aca cct     1008
Leu Leu Glu Leu Asp Lys Pro Leu Ile Leu Asn Ser Tyr Val Thr Pro
                325                 330                 335

atc tgt gtt gcc aat agg gaa tat aca aat atc ttc ctc aag ttt ggt     1056
Ile Cys Val Ala Asn Arg Glu Tyr Thr Asn Ile Phe Leu Lys Phe Gly
            340                 345                 350

tct ggc tat gtc agt ggc tgg gga aaa gtc ttc aac aaa ggg aga cag     1104
Ser Gly Tyr Val Ser Gly Trp Gly Lys Val Phe Asn Lys Gly Arg Gln
        355                 360                 365

gct tcc att ctt cag tac ctt aga gtt cca ctg gtg gat aga gcc aca     1152
Ala Ser Ile Leu Gln Tyr Leu Arg Val Pro Leu Val Asp Arg Ala Thr
    370                 375                 380

tgc ctt agg tcc aca aca ttc act atc tat aac aac atg ttc tgt gca     1200
Cys Leu Arg Ser Thr Thr Phe Thr Ile Tyr Asn Asn Met Phe Cys Ala
385                 390                 395                 400

ggc tac cgt gaa gga ggc aaa gat tcg tgt gaa gga gat agt ggg gga     1248
Gly Tyr Arg Glu Gly Gly Lys Asp Ser Cys Glu Gly Asp Ser Gly Gly

405                 410                 415

ccc cat gtt act gaa gta gaa ggg aca agt ttc tta act ggc att att      1296
Pro His Val Thr Glu Val Glu Gly Thr Ser Phe Leu Thr Gly Ile Ile
            420                 425                 430

agc tgg ggt gaa gaa tgt gca atg aaa ggc aaa tat gga ata tat act      1344
Ser Trp Gly Glu Glu Cys Ala Met Lys Gly Lys Tyr Gly Ile Tyr Thr
        435                 440                 445

aag gtt tcc cgg tac gtc aac tgg att aag gaa aaa aca aag cta act      1392
Lys Val Ser Arg Tyr Val Asn Trp Ile Lys Glu Lys Thr Lys Leu Thr
    450                 455                 460

taa                                                                  1395


<210>  23
<211>  464
<212>  PRT
<213>  Mus musculus

<400>  23

Met Ala Glu Ser Pro Ala Leu Ile Thr Ile Phe Leu Leu Gly Tyr Leu
1               5                   10                  15


Leu Ser Thr Glu Cys Ala Val Phe Leu Asp Arg Glu Asn Ala Thr Lys
            20                  25                  30


Ile Leu Thr Arg Pro Lys Arg Tyr Asn Ser Gly Lys Leu Glu Glu Phe
        35                  40                  45


Val Arg Gly Asn Leu Glu Arg Glu Cys Ile Glu Glu Arg Cys Ser Phe
    50                  55                  60


Glu Glu Ala Arg Glu Val Phe Glu Asn Thr Glu Lys Thr Thr Glu Phe
65                  70                  75                  80


Trp Lys Gln Tyr Val Asp Gly Asp Gln Cys Glu Ser Asn Pro Cys Leu
                85                  90                  95


Asn Gly Gly Ile Cys Lys Asp Asp Ile Ser Ser Tyr Glu Cys Trp Cys
            100                 105                 110


Gln Val Gly Phe Glu Gly Arg Asn Cys Glu Leu Asp Ala Thr Cys Asn
        115                 120                 125


Ile Lys Asn Gly Arg Cys Lys Gln Phe Cys Lys Asn Ser Pro Asp Asn
    130                 135                 140


Lys Val Ile Cys Ser Cys Thr Glu Gly Tyr Gln Leu Ala Glu Asp Gln
145                 150                 155                 160


Lys Ser Cys Glu Pro Thr Val Pro Phe Pro Cys Gly Arg Ala Ser Ile

                165                 170                 175

Ser Tyr Ser Ser Lys Lys Ile Thr Arg Ala Glu Thr Val Phe Ser Asn
            180                 185                 190

Met Asp Tyr Glu Asn Ser Thr Glu Ala Val Phe Ile Gln Asp Asp Ile
        195                 200                 205

Thr Asp Gly Ala Ile Leu Asn Asn Val Thr Glu Ser Ser Glu Ser Leu
    210                 215                 220

Asn Asp Phe Thr Arg Val Val Gly Gly Glu Asn Ala Lys Pro Gly Gln
225                 230                 235                 240

Ile Pro Trp Gln Val Ile Leu Asn Gly Glu Ile Glu Ala Phe Cys Gly
                245                 250                 255

Gly Ala Ile Ile Asn Glu Lys Trp Ile Val Thr Ala Ala His Cys Leu
            260                 265                 270

Lys Pro Gly Asp Lys Ile Glu Val Val Ala Gly Glu Tyr Asn Ile Asp
        275                 280                 285

Lys Lys Glu Asp Thr Glu Gln Arg Arg Asn Val Ile Arg Thr Ile Pro
    290                 295                 300

His His Gln Tyr Asn Ala Thr Ile Asn Lys Tyr Ser His Asp Ile Ala
305                 310                 315                 320

Leu Leu Glu Leu Asp Lys Pro Leu Ile Leu Asn Ser Tyr Val Thr Pro
                325                 330                 335

Ile Cys Val Ala Asn Arg Glu Tyr Thr Asn Ile Phe Leu Lys Phe Gly
            340                 345                 350

Ser Gly Tyr Val Ser Gly Trp Gly Lys Val Phe Asn Lys Gly Arg Gln
        355                 360                 365

Ala Ser Ile Leu Gln Tyr Leu Arg Val Pro Leu Val Asp Arg Ala Thr
    370                 375                 380

Cys Leu Arg Ser Thr Thr Phe Thr Ile Tyr Asn Asn Met Phe Cys Ala
385                 390                 395                 400

Gly Tyr Arg Glu Gly Gly Lys Asp Ser Cys Glu Gly Asp Ser Gly Gly
                405                 410                 415

Pro His Val Thr Glu Val Glu Gly Thr Ser Phe Leu Thr Gly Ile Ile
            420                 425                 430

Ser Trp Gly Glu Glu Cys Ala Met Lys Gly Lys Tyr Gly Ile Tyr Thr
        435                 440                 445

Lys Val Ser Arg Tyr Val Asn Trp Ile Lys Glu Lys Thr Lys Leu Thr
    450                 455                 460

<210>  24
<211>  102
<212>  DNA
<213>  Mus musculus

<220>
<221>  CDS
<222>  (1)..(102)

<400>  24
tgt gaa tca aat cct tgt tta aat ggt gga ata tgc aag gat gat att       48
Cys Glu Ser Asn Pro Cys Leu Asn Gly Gly Ile Cys Lys Asp Asp Ile
1               5                   10                  15

agt tcc tat gaa tgc tgg tgc caa gtt gga ttt gaa gga agg aac tgt       96
Ser Ser Tyr Glu Cys Trp Cys Gln Val Gly Phe Glu Gly Arg Asn Cys
            20                  25                  30

gaa tta                                                               102
Glu Leu

<210>  25
<211>  34
<212>  PRT
<213>  Mus musculus

<400>  25

Cys Glu Ser Asn Pro Cys Leu Asn Gly Gly Ile Cys Lys Asp Asp Ile
1               5                   10                  15

Ser Ser Tyr Glu Cys Trp Cys Gln Val Gly Phe Glu Gly Arg Asn Cys
            20                  25                  30

Glu Leu

<210>  26
<211>  156
<212>  DNA
<213>  Mus musculus

<220>
<221>  CDS
<222>  (1)..(156)

<400>  26

tgc aac ccg aac ccc tgt gaa aat ggt ggc atc tgt ctg tca gga ctg 48
Cys Asn Pro Asn Pro Cys Glu Asn Gly Gly Ile Cys Leu Ser Gly Leu
1               5                   10                  15

gct gat gat tcc ttt tcc tgt gag tgt cca gaa ggc ttc gca ggt ccg 96
Ala Asp Asp Ser Phe Ser Cys Glu Cys Pro Glu Gly Phe Ala Gly Pro
            20                  25                  30

aac tgc tct agt gtt gtg gag gtt gca tca gat gaa gaa aag cct act 144
Asn Cys Ser Ser Val Val Glu Val Ala Ser Asp Glu Glu Lys Pro Thr
        35                  40                  45

tca gca ggt ccc 156
Ser Ala Gly Pro
    50

<210> 27
<211> 52
<212> PRT
<213> Mus musculus

<400> 27

Cys Asn Pro Asn Pro Cys Glu Asn Gly Gly Ile Cys Leu Ser Gly Leu
1               5                   10                  15

Ala Asp Asp Ser Phe Ser Cys Glu Cys Pro Glu Gly Phe Ala Gly Pro
            20                  25                  30

Asn Cys Ser Ser Val Val Glu Val Ala Ser Asp Glu Glu Lys Pro Thr
        35                  40                  45

Ser Ala Gly Pro
    50

<210> 28
<211> 135
<212> DNA
<213> Mus musculus

<220>
<221> CDS
<222> (1)..(135)

<400> 28
tgc atc cct aac cca tgc cat aac gga gga acc tgt gag ata agc gaa 48
Cys Ile Pro Asn Pro Cys His Asn Gly Gly Thr Cys Glu Ile Ser Glu
1               5                   10                  15

gcc tat cga gga gac aca ttc ata ggc tat gtt tgt aaa tgt cct cgg 96
Ala Tyr Arg Gly Asp Thr Phe Ile Gly Tyr Val Cys Lys Cys Pro Arg
            20                  25                  30

gga ttt aat ggg att cac tgt cag cac aat ata aat gaa 135
Gly Phe Asn Gly Ile His Cys Gln His Asn Ile Asn Glu
        35                  40                  45

<210> 29

<211>  45
<212>  PRT
<213>  Mus musculus

<400>  29

Cys Ile Pro Asn Pro Cys His Asn Gly Gly Thr Cys Glu Ile Ser Glu
1               5                   10                  15

Ala Tyr Arg Gly Asp Thr Phe Ile Gly Tyr Val Cys Lys Cys Pro Arg
            20                  25                  30

Gly Phe Asn Gly Ile His Cys Gln His Asn Ile Asn Glu
        35                  40                  45

<210>  30
<211>  972
<212>  DNA
<213>  Mus musculus

<220>
<221>  CDS
<222>  (1)..(972)

<400>  30
tgc tct ggg cca ttg gga atc gaa ggt ggg atc ata tct aat cag caa      48
Cys Ser Gly Pro Leu Gly Ile Glu Gly Gly Ile Ile Ser Asn Gln Gln
1               5                   10                  15

atc aca gct tca tct act cac cga gct ctt ttt gga ctc cgg aag tgg      96
Ile Thr Ala Ser Ser Thr His Arg Ala Leu Phe Gly Leu Arg Lys Trp
            20                  25                  30

tat ccc tac tat gct cga ctt aat aag aag ggc ctt ata aat gcc tgg      144
Tyr Pro Tyr Tyr Ala Arg Leu Asn Lys Lys Gly Leu Ile Asn Ala Trp
        35                  40                  45

aca gct gct gaa aat gac aga tgg cca tgg att cag ata aat ttg caa      192
Thr Ala Ala Glu Asn Asp Arg Trp Pro Trp Ile Gln Ile Asn Leu Gln
    50                  55                  60

aga aaa atg aga gtc act ggt gtt att acc caa gga gca aaa agg att      240
Arg Lys Met Arg Val Thr Gly Val Ile Thr Gln Gly Ala Lys Arg Ile
65                  70                  75                  80

gga agc cca gag tac ata aaa tcc tac aaa att gcc tac agc aat gac      288
Gly Ser Pro Glu Tyr Ile Lys Ser Tyr Lys Ile Ala Tyr Ser Asn Asp
                85                  90                  95

ggg aag acc tgg gca atg tac aaa gta aaa ggc acc aat gaa gag atg      336
Gly Lys Thr Trp Ala Met Tyr Lys Val Lys Gly Thr Asn Glu Glu Met
            100                 105                 110

gtc ttt cgt gga aat gtt gat aac aac aca cca tat gct aat tct ttc      384
Val Phe Arg Gly Asn Val Asp Asn Asn Thr Pro Tyr Ala Asn Ser Phe
        115                 120                 125

aca ccc cca atc aaa gct cag tat gta aga ctc tac ccc caa att tgt      432
Thr Pro Pro Ile Lys Ala Gln Tyr Val Arg Leu Tyr Pro Gln Ile Cys
    130                 135                 140

cga agg cat tgt act tta aga atg gaa ctt ctt ggc tgt gag ctc tca 480
Arg Arg His Cys Thr Leu Arg Met Glu Leu Leu Gly Cys Glu Leu Ser
145                 150                 155                 160

ggc tgt tca gaa cct ttg ggg atg aaa tca ggg cat ata caa gac tac 528
Gly Cys Ser Glu Pro Leu Gly Met Lys Ser Gly His Ile Gln Asp Tyr
                165                 170                 175

cag atc act gcc tcc agc gtc ttc aga aca ctc aac atg gac atg ttt 576
Gln Ile Thr Ala Ser Ser Val Phe Arg Thr Leu Asn Met Asp Met Phe
            180                 185                 190

act tgg gaa cca agg aaa gcc agg ctg gac aag caa ggc aaa gta aat 624
Thr Trp Glu Pro Arg Lys Ala Arg Leu Asp Lys Gln Gly Lys Val Asn
        195                 200                 205

gcc tgg act tcc ggc cat aac gac cag tca caa tgg tta cag gtt gat 672
Ala Trp Thr Ser Gly His Asn Asp Gln Ser Gln Trp Leu Gln Val Asp
    210                 215                 220

ctt ctt gtc cct act aag gtg aca ggc atc att aca caa gga gct aaa 720
Leu Leu Val Pro Thr Lys Val Thr Gly Ile Ile Thr Gln Gly Ala Lys
225                 230                 235                 240

gat ttt ggt cac gtg cag ttt gtt ggg tca tac aaa cta gct tac agc 768
Asp Phe Gly His Val Gln Phe Val Gly Ser Tyr Lys Leu Ala Tyr Ser
                245                 250                 255

aat gat gga gaa cac tgg atg gtg cac cag gat gaa aaa cag agg aaa 816
Asn Asp Gly Glu His Trp Met Val His Gln Asp Glu Lys Gln Arg Lys
            260                 265                 270

gac aag gtt ttt caa ggc aat ttt gac aat gac act cac agg aaa aat 864
Asp Lys Val Phe Gln Gly Asn Phe Asp Asn Asp Thr His Arg Lys Asn
        275                 280                 285

gtc atc gac cct ccc atc tat gca cga ttc ata aga atc ctt cct tgg 912
Val Ile Asp Pro Pro Ile Tyr Ala Arg Phe Ile Arg Ile Leu Pro Trp
    290                 295                 300

tcc tgg tat gga agg atc act ctg cgg tca gag ctg ctg ggc tgc gca 960
Ser Trp Tyr Gly Arg Ile Thr Leu Arg Ser Glu Leu Leu Gly Cys Ala
305                 310                 315                 320

gag gag gaa tga 972
Glu Glu Glu

<210> 31
<211> 323
<212> PRT
<213> Mus musculus

<400> 31

Cys Ser Gly Pro Leu Gly Ile Glu Gly Gly Ile Ile Ser Asn Gln Gln
1               5                   10                  15

Ile Thr Ala Ser Ser Thr His Arg Ala Leu Phe Gly Leu Arg Lys Trp
            20                  25                  30

Tyr Pro Tyr Tyr Ala Arg Leu Asn Lys Lys Gly Leu Ile Asn Ala Trp

35                  40                  45

Thr Ala Ala Glu Asn Asp Arg Trp Pro Trp Ile Gln Ile Asn Leu Gln
    50                  55                  60

Arg Lys Met Arg Val Thr Gly Val Ile Thr Gln Gly Ala Lys Arg Ile
65                  70                  75                  80

Gly Ser Pro Glu Tyr Ile Lys Ser Tyr Lys Ile Ala Tyr Ser Asn Asp
                85                  90                  95

Gly Lys Thr Trp Ala Met Tyr Lys Val Lys Gly Thr Asn Glu Glu Met
            100                 105                 110

Val Phe Arg Gly Asn Val Asp Asn Asn Thr Pro Tyr Ala Asn Ser Phe
        115                 120                 125

Thr Pro Pro Ile Lys Ala Gln Tyr Val Arg Leu Tyr Pro Gln Ile Cys
    130                 135                 140

Arg Arg His Cys Thr Leu Arg Met Glu Leu Leu Gly Cys Glu Leu Ser
145                 150                 155                 160

Gly Cys Ser Glu Pro Leu Gly Met Lys Ser Gly His Ile Gln Asp Tyr
                165                 170                 175

Gln Ile Thr Ala Ser Ser Val Phe Arg Thr Leu Asn Met Asp Met Phe
            180                 185                 190

Thr Trp Glu Pro Arg Lys Ala Arg Leu Asp Lys Gln Gly Lys Val Asn
        195                 200                 205

Ala Trp Thr Ser Gly His Asn Asp Gln Ser Gln Trp Leu Gln Val Asp
    210                 215                 220

Leu Leu Val Pro Thr Lys Val Thr Gly Ile Ile Thr Gln Gly Ala Lys
225                 230                 235                 240

Asp Phe Gly His Val Gln Phe Val Gly Ser Tyr Lys Leu Ala Tyr Ser
                245                 250                 255

Asn Asp Gly Glu His Trp Met Val His Gln Asp Glu Lys Gln Arg Lys
            260                 265                 270

Asp Lys Val Phe Gln Gly Asn Phe Asp Asn Asp Thr His Arg Lys Asn
        275                 280                 285

Val Ile Asp Pro Pro Ile Tyr Ala Arg Phe Ile Arg Ile Leu Pro Trp
    290                 295                 300

Ser Trp Tyr Gly Arg Ile Thr Leu Arg Ser Glu Leu Leu Gly Cys Ala
305                 310                 315                 320

Glu Glu Glu

<210>  32
<211>  2303
<212>  DNA
<213>  Mus musculus

<220>
<221>  CDS
<222>  (619)..(2061)

<400>  32
gaattccggt taactgagga caaagggtaa tgcagaagtg atatttgatt tccattctca      60

ttcccagtgg ccttgatatt taaactgatt cctgccacca ggtccttggg ccaccctgtc     120

cctgcgtctc atatttctgc atgctgcttt gtttgtatat agtgcgctcc tggcctcagg     180

ctcgctcccc tccagctctc gcttcattgt tctccaagtc agaagccccc gcatccgccg     240

cgcagcagcg tgagccgtag tcactgctgg ccgcttcgcc tgcgtgcgcg cacggaaatc     300

ggggagccag gaacccaagg agccgccgtc cgcccgctgt gcctctgcta gaccactcgc     360

agccccagcc tctctcaagc gcacccacct ccgcgcaccc cagctcaggc gaagctggag     420

tgagggtgaa tcaccctttc tctagggcca ccactctttt atcgcccttc ccaagatttg     480

agaagcgctg cgggaggaaa gacgtcctct tgatctctga cagggcgggg tttactgctg     540

tcctgcaggc gcgcctcgcc tactgtgccc tccgctacga ccccggacca gcccaggtca     600

cgtccgtgag aagggatc atg aag cac ttg gta gca gcc tgg ctt ttg gtt      651
                    Met Lys His Leu Val Ala Ala Trp Leu Leu Val
                    1               5                   10

gga ctc agc ctc ggg gtg ccc cag ttc ggc aaa ggt gac att tgc aac      699
Gly Leu Ser Leu Gly Val Pro Gln Phe Gly Lys Gly Asp Ile Cys Asn
            15                  20                  25

ccg aac ccc tgt gaa aat ggt ggc atc tgt ctg tca gga ctg gct gat      747
Pro Asn Pro Cys Glu Asn Gly Gly Ile Cys Leu Ser Gly Leu Ala Asp
        30                  35                  40

gat tcc ttt tcc tgt gag tgt cca gaa ggc ttc gca ggt ccg aac tgc      795
Asp Ser Phe Ser Cys Glu Cys Pro Glu Gly Phe Ala Gly Pro Asn Cys
    45                  50                  55

tct agt gtt gtg gag gtt gca tca gat gaa gaa aag cct act tca gca      843
Ser Ser Val Val Glu Val Ala Ser Asp Glu Glu Lys Pro Thr Ser Ala
60                  65                  70                  75

ggt ccc tgc atc cct aac cca tgc cat aac gga gga acc tgt gag ata      891
Gly Pro Cys Ile Pro Asn Pro Cys His Asn Gly Gly Thr Cys Glu Ile
                80                  85                  90

agc gaa gcc tat cga gga gac aca ttc ata ggc tat gtt tgt aaa tgt     939
Ser Glu Ala Tyr Arg Gly Asp Thr Phe Ile Gly Tyr Val Cys Lys Cys
            95                  100                 105

cct cgg gga ttt aat ggg att cac tgt cag cac aat ata aat gaa tgt     987
Pro Arg Gly Phe Asn Gly Ile His Cys Gln His Asn Ile Asn Glu Cys
        110                 115                 120

gaa gct gag cct tgc aga aat ggc gga ata tgt acc gaa ctt gtt gct     1035
Glu Ala Glu Pro Cys Arg Asn Gly Gly Ile Cys Thr Glu Leu Val Ala
    125                 130                 135

aac tac tct tgt gaa tgc cca gga gaa ttt atg gga cga aat tgt caa     1083
Asn Tyr Ser Cys Glu Cys Pro Gly Glu Phe Met Gly Arg Asn Cys Gln
140                 145                 150                 155

tat aaa tgc tct ggg cca ttg gga atc gaa ggt ggg atc ata tct aat     1131
Tyr Lys Cys Ser Gly Pro Leu Gly Ile Glu Gly Gly Ile Ile Ser Asn
                160                 165                 170

cag caa atc aca gct tca tct act cac cga gct ctt ttt gga ctc cgg     1179
Gln Gln Ile Thr Ala Ser Ser Thr His Arg Ala Leu Phe Gly Leu Arg
            175                 180                 185

aag tgg tat ccc tac tat gct cga ctt aat aag aag ggc ctt ata aat     1227
Lys Trp Tyr Pro Tyr Tyr Ala Arg Leu Asn Lys Lys Gly Leu Ile Asn
        190                 195                 200

gcc tgg aca gct gct gaa aat gac aga tgg cca tgg att cag ata aat     1275
Ala Trp Thr Ala Ala Glu Asn Asp Arg Trp Pro Trp Ile Gln Ile Asn
    205                 210                 215

ttg caa aga aaa atg aga gtc act ggt gtt att acc caa gga gca aaa     1323
Leu Gln Arg Lys Met Arg Val Thr Gly Val Ile Thr Gln Gly Ala Lys
220                 225                 230                 235

agg att gga agc cca gag tac ata aaa tcc tac aaa att gcc tac agc     1371
Arg Ile Gly Ser Pro Glu Tyr Ile Lys Ser Tyr Lys Ile Ala Tyr Ser
                240                 245                 250

aat gac ggg aag acc tgg gca atg tac aaa gta aaa ggc acc aat gaa     1419
Asn Asp Gly Lys Thr Trp Ala Met Tyr Lys Val Lys Gly Thr Asn Glu
            255                 260                 265

gag atg gtc ttt cgt gga aat gtt gat aac aac aca cca tat gct aat     1467
Glu Met Val Phe Arg Gly Asn Val Asp Asn Asn Thr Pro Tyr Ala Asn
        270                 275                 280

tct ttc aca ccc cca atc aaa gct cag tat gta aga ctc tac ccc caa     1515
Ser Phe Thr Pro Pro Ile Lys Ala Gln Tyr Val Arg Leu Tyr Pro Gln
    285                 290                 295

att tgt cga agg cat tgt act tta aga atg gaa ctt ctt ggc tgt gag     1563
Ile Cys Arg Arg His Cys Thr Leu Arg Met Glu Leu Leu Gly Cys Glu
300                 305                 310                 315

ctc tca ggc tgt tca gaa cct ttg ggg atg aaa tca ggg cat ata caa     1611
Leu Ser Gly Cys Ser Glu Pro Leu Gly Met Lys Ser Gly His Ile Gln
                320                 325                 330

gac tac cag atc act gcc tcc agc gtc ttc aga aca ctc aac atg gac     1659
Asp Tyr Gln Ile Thr Ala Ser Ser Val Phe Arg Thr Leu Asn Met Asp
            335                 340                 345

atg ttt act tgg gaa cca agg aaa gcc agg ctg gac aag caa ggc aaa     1707

Met Phe Thr Trp Glu Pro Arg Lys Ala Arg Leu Asp Lys Gln Gly Lys
        350                 355                 360

gta aat gcc tgg act tcc ggc cat aac gac cag tca caa tgg tta cag      1755
Val Asn Ala Trp Thr Ser Gly His Asn Asp Gln Ser Gln Trp Leu Gln
    365                 370                 375

gtt gat ctt ctt gtc cct act aag gtg aca ggc atc att aca caa gga      1803
Val Asp Leu Leu Val Pro Thr Lys Val Thr Gly Ile Ile Thr Gln Gly
380                 385                 390                 395

gct aaa gat ttt ggt cac gtg cag ttt gtt ggg tca tac aaa cta gct      1851
Ala Lys Asp Phe Gly His Val Gln Phe Val Gly Ser Tyr Lys Leu Ala
                400                 405                 410

tac agc aat gat gga gaa cac tgg atg gtg cac cag gat gaa aaa cag      1899
Tyr Ser Asn Asp Gly Glu His Trp Met Val His Gln Asp Glu Lys Gln
            415                 420                 425

agg aaa gac aag gtt ttt caa ggc aat ttt gac aat gac act cac agg      1947
Arg Lys Asp Lys Val Phe Gln Gly Asn Phe Asp Asn Asp Thr His Arg
        430                 435                 440

aaa aat gtc atc gac cct ccc atc tat gca cga ttc ata aga atc ctt      1995
Lys Asn Val Ile Asp Pro Pro Ile Tyr Ala Arg Phe Ile Arg Ile Leu
    445                 450                 455

cct tgg tcc tgg tat gga agg atc act ctg cgg tca gag ctg ctg ggc      2043
Pro Trp Ser Trp Tyr Gly Arg Ile Thr Leu Arg Ser Glu Leu Leu Gly
460                 465                 470                 475

tgc gca gag gag gaa tga agtgcggggc cgcacatccc acaatgcttt             2091
Cys Ala Glu Glu Glu
                480

tctttatttt cctataagta tctccacgaa atgaactgtg tgaagctgat ggaaactgca   2151

tttgtttttt tcaaagtgtt caaattatgg taggctactg actgtctttt taggagttct   2211

aagcttgcct ttttaataat ttaatttggt ttcctttgct caactctctt atgtaatatc    2271

acactgtctg tgagttactc ttcttgttct ct                                 2303


<210>  33
<211>  480
<212>  PRT
<213>  Mus musculus

<400>  33

Met Lys His Leu Val Ala Ala Trp Leu Leu Val Gly Leu Ser Leu Gly
1               5                   10                  15


Val Pro Gln Phe Gly Lys Gly Asp Ile Cys Asn Pro Asn Pro Cys Glu
            20                  25                  30


Asn Gly Gly Ile Cys Leu Ser Gly Leu Ala Asp Asp Ser Phe Ser Cys
        35                  40                  45


Glu Cys Pro Glu Gly Phe Ala Gly Pro Asn Cys Ser Ser Val Val Glu
    50                  55                  60

Val Ala Ser Asp Glu Glu Lys Pro Thr Ser Ala Gly Pro Cys Ile Pro
65                  70                  75                  80

Asn Pro Cys His Asn Gly Gly Thr Cys Glu Ile Ser Glu Ala Tyr Arg
                85                  90                  95

Gly Asp Thr Phe Ile Gly Tyr Val Cys Lys Cys Pro Arg Gly Phe Asn
            100                 105                 110

Gly Ile His Cys Gln His Asn Ile Asn Glu Cys Glu Ala Glu Pro Cys
        115                 120                 125

Arg Asn Gly Gly Ile Cys Thr Glu Leu Val Ala Asn Tyr Ser Cys Glu
    130                 135                 140

Cys Pro Gly Glu Phe Met Gly Arg Asn Cys Gln Tyr Lys Cys Ser Gly
145                 150                 155                 160

Pro Leu Gly Ile Glu Gly Gly Ile Ile Ser Asn Gln Gln Ile Thr Ala
                165                 170                 175

Ser Ser Thr His Arg Ala Leu Phe Gly Leu Arg Lys Trp Tyr Pro Tyr
            180                 185                 190

Tyr Ala Arg Leu Asn Lys Lys Gly Leu Ile Asn Ala Trp Thr Ala Ala
        195                 200                 205

Glu Asn Asp Arg Trp Pro Trp Ile Gln Ile Asn Leu Gln Arg Lys Met
    210                 215                 220

Arg Val Thr Gly Val Ile Thr Gln Gly Ala Lys Arg Ile Gly Ser Pro
225                 230                 235                 240

Glu Tyr Ile Lys Ser Tyr Lys Ile Ala Tyr Ser Asn Asp Gly Lys Thr
                245                 250                 255

Trp Ala Met Tyr Lys Val Lys Gly Thr Asn Glu Glu Met Val Phe Arg
            260                 265                 270

Gly Asn Val Asp Asn Asn Thr Pro Tyr Ala Asn Ser Phe Thr Pro Pro
        275                 280                 285

Ile Lys Ala Gln Tyr Val Arg Leu Tyr Pro Gln Ile Cys Arg Arg His
    290                 295                 300

Cys Thr Leu Arg Met Glu Leu Leu Gly Cys Glu Leu Ser Gly Cys Ser
305                 310                 315                 320

Glu Pro Leu Gly Met Lys Ser Gly His Ile Gln Asp Tyr Gln Ile Thr
                325                 330                 335

Ala Ser Ser Val Phe Arg Thr Leu Asn Met Asp Met Phe Thr Trp Glu
            340                 345                 350

Pro Arg Lys Ala Arg Leu Asp Lys Gln Gly Lys Val Asn Ala Trp Thr
        355                 360                 365

Ser Gly His Asn Asp Gln Ser Gln Trp Leu Gln Val Asp Leu Leu Val
    370                 375                 380

Pro Thr Lys Val Thr Gly Ile Ile Thr Gln Gly Ala Lys Asp Phe Gly
385                 390                 395                 400

His Val Gln Phe Val Gly Ser Tyr Lys Leu Ala Tyr Ser Asn Asp Gly
                405                 410                 415

Glu His Trp Met Val His Gln Asp Glu Lys Gln Arg Lys Asp Lys Val
            420                 425                 430

Phe Gln Gly Asn Phe Asp Asn Asp Thr His Arg Lys Asn Val Ile Asp
        435                 440                 445

Pro Pro Ile Tyr Ala Arg Phe Ile Arg Ile Leu Pro Trp Ser Trp Tyr
    450                 455                 460

Gly Arg Ile Thr Leu Arg Ser Glu Leu Leu Gly Cys Ala Glu Glu Glu
465                 470                 475                 480


<210>  34
<211>  105
<212>  DNA
<213>  Mus musculus


<220>
<221>  CDS
<222>  (1)..(105)

<400>  34
tgt gaa gct gag cct tgc aga aat ggc gga ata tgt acc gaa ctt gtt       48
Cys Glu Ala Glu Pro Cys Arg Asn Gly Gly Ile Cys Thr Glu Leu Val
1               5                   10                  15

gct aac tac tct tgt gaa tgc cca gga gaa ttt atg gga cga aat tgt       96
Ala Asn Tyr Ser Cys Glu Cys Pro Gly Glu Phe Met Gly Arg Asn Cys
            20                  25                  30

caa tat aaa                                                           105
Gln Tyr Lys
        35

<210>  35
<211>  35
<212>  PRT
<213>  Mus musculus

<400>  35

Cys Glu Ala Glu Pro Cys Arg Asn Gly Gly Ile Cys Thr Glu Leu Val
1               5                   10                  15

Ala Asn Tyr Ser Cys Glu Cys Pro Gly Glu Phe Met Gly Arg Asn Cys
            20                  25                  30

Gln Tyr Lys
        35

<210>  36
<211>  105
<212>  DNA
<213>  Mus musculus

<220>
<221>  CDS
<222>  (1)..(105)

<400>  36
tgt gaa gct gag cct tgc aga aat ggc gga ata tgt acc aac ctt gtt       48
Cys Glu Ala Glu Pro Cys Arg Asn Gly Gly Ile Cys Thr Asn Leu Val
1               5                   10                  15

gct aac tac tct tgt gaa tgc cca gga gaa ttt atg gga cga aat tgt       96
Ala Asn Tyr Ser Cys Glu Cys Pro Gly Glu Phe Met Gly Arg Asn Cys
            20                  25                  30

caa tat aaa                                                           105
Gln Tyr Lys
        35

<210>  37
<211>  35
<212>  PRT
<213>  Mus musculus

<400>  37

Cys Glu Ala Glu Pro Cys Arg Asn Gly Gly Ile Cys Thr Asn Leu Val
1               5                   10                  15

Ala Asn Tyr Ser Cys Glu Cys Pro Gly Glu Phe Met Gly Arg Asn Cys
            20                  25                  30

Gln Tyr Lys
        35

<210>  38
<211>  105
<212>  DNA

<213>  Mus musculus

<220>
<221>  CDS
<222>  (1)..(105)

<400>  38
tgt gaa gct gag cct tgc aga aat ggc gga ata tgt acc gac ctt gtt        48
Cys Glu Ala Glu Pro Cys Arg Asn Gly Gly Ile Cys Thr Asp Leu Val
1               5                   10                  15

gct aac ttc tct tgt gaa tgc cca gga gaa ttt atg gga cga aat tgt        96
Ala Asn Phe Ser Cys Glu Cys Pro Gly Glu Phe Met Gly Arg Asn Cys
            20                  25                  30

caa tat aaa                                                            105
Gln Tyr Lys
        35

<210>  39
<211>  35
<212>  PRT
<213>  Mus musculus

<400>  39

Cys Glu Ala Glu Pro Cys Arg Asn Gly Gly Ile Cys Thr Asp Leu Val
1               5                   10                  15

Ala Asn Phe Ser Cys Glu Cys Pro Gly Glu Phe Met Gly Arg Asn Cys
            20                  25                  30

Gln Tyr Lys
        35

<210>  40
<211>  12
<212>  PRT
<213>  Artificial

<220>
<223>  peptide

<220>
<221>  MISC_FEATURE
<222>  (2)..(2)
<223>  Xaa can be any naturally occurring amino acid

<220>
<221>  MISC_FEATURE
<222>  (3)..(3)
<223>  Xaa represents Asp or Asn

<220>
<221>  MISC_FEATURE
<222>  (4)..(7)
<223>  Xaa can be any naturally occurring amino acid

<220>

<221>  MISC_FEATURE
<222>  (8)..(8)
<223>  Xaa represents Phe or Tyr

<220>
<221>  MISC_FEATURE
<222>  (9)..(9)
<223>  Xaa can be any naturally occurring amino acid

<220>
<221>  MISC_FEATURE
<222>  (11)..(11)
<223>  Xaa can be any naturally occurring amino acid

<400>  40

Cys Xaa Xaa Xaa Xaa Xaa Xaa Xaa Xaa Cys Xaa Cys
1               5                   10


<210>  41
<211>  591
<212>  DNA
<213>  Mus musculus


<220>
<221>  CDS
<222>  (1)..(591)

<400>  41
tgt gaa gct gag cct tgc aga aat ggc gga ata tgt acc gac ctt gtt       48
Cys Glu Ala Glu Pro Cys Arg Asn Gly Gly Ile Cys Thr Asp Leu Val
1               5                   10                  15

gct aac tac tct tgt gaa tgc cca gga gaa ttt atg gga cga aat tgt       96
Ala Asn Tyr Ser Cys Glu Cys Pro Gly Glu Phe Met Gly Arg Asn Cys
            20                  25                  30

caa tat aaa tgc tct ggg cca ttg gga atc gaa ggt ggg atc ata tct      144
Gln Tyr Lys Cys Ser Gly Pro Leu Gly Ile Glu Gly Gly Ile Ile Ser
        35                  40                  45

aat cag caa atc aca gct tca tct act cac cga gct ctt ttt gga ctc      192
Asn Gln Gln Ile Thr Ala Ser Ser Thr His Arg Ala Leu Phe Gly Leu
    50                  55                  60

cgg aag tgg tat ccc tac tat gct cga ctt aat aag aag ggc ctt ata      240
Arg Lys Trp Tyr Pro Tyr Tyr Ala Arg Leu Asn Lys Lys Gly Leu Ile
65                  70                  75                  80

aat gcc tgg aca gct gct gaa aat gac aga tgg cca tgg att cag ata      288
Asn Ala Trp Thr Ala Ala Glu Asn Asp Arg Trp Pro Trp Ile Gln Ile
                85                  90                  95

aat ttg caa aga aaa atg aga gtc act ggt gtt att acc caa gga gca      336
Asn Leu Gln Arg Lys Met Arg Val Thr Gly Val Ile Thr Gln Gly Ala
            100                 105                 110

aaa agg att gga agc cca gag tac ata aaa tcc tac aaa att gcc tac      384
Lys Arg Ile Gly Ser Pro Glu Tyr Ile Lys Ser Tyr Lys Ile Ala Tyr
        115                 120                 125

agc aat gac ggg aag acc tgg gca atg tac aaa gta aaa ggc acc aat      432
Ser Asn Asp Gly Lys Thr Trp Ala Met Tyr Lys Val Lys Gly Thr Asn

130                 135                 140

gaa gag atg gtc ttt cgt gga aat gtt gat aac aac aca cca tat gct       480
Glu Glu Met Val Phe Arg Gly Asn Val Asp Asn Asn Thr Pro Tyr Ala
145                 150                 155                 160

aat tct ttc aca ccc cca atc aaa gct cag tat gta aga ctc tac ccc       528
Asn Ser Phe Thr Pro Pro Ile Lys Ala Gln Tyr Val Arg Leu Tyr Pro
                165                 170                 175

caa att tgt cga agg cat tgt act tta aga atg gaa ctt ctt ggc tgt       576
Gln Ile Cys Arg Arg His Cys Thr Leu Arg Met Glu Leu Leu Gly Cys
            180                 185                 190

gag ctc tca ggc tgt                                                   591
Glu Leu Ser Gly Cys
        195

<210>  42
<211>  197
<212>  PRT
<213>  Mus musculus

<400>  42

Cys Glu Ala Glu Pro Cys Arg Asn Gly Gly Ile Cys Thr Asp Leu Val
1               5                   10                  15

Ala Asn Tyr Ser Cys Glu Cys Pro Gly Glu Phe Met Gly Arg Asn Cys
            20                  25                  30

Gln Tyr Lys Cys Ser Gly Pro Leu Gly Ile Glu Gly Gly Ile Ile Ser
        35                  40                  45

Asn Gln Gln Ile Thr Ala Ser Ser Thr His Arg Ala Leu Phe Gly Leu
    50                  55                  60

Arg Lys Trp Tyr Pro Tyr Tyr Ala Arg Leu Asn Lys Lys Gly Leu Ile
65                  70                  75                  80

Asn Ala Trp Thr Ala Ala Glu Asn Asp Arg Trp Pro Trp Ile Gln Ile
                85                  90                  95

Asn Leu Gln Arg Lys Met Arg Val Thr Gly Val Ile Thr Gln Gly Ala
            100                 105                 110

Lys Arg Ile Gly Ser Pro Glu Tyr Ile Lys Ser Tyr Lys Ile Ala Tyr
        115                 120                 125

Ser Asn Asp Gly Lys Thr Trp Ala Met Tyr Lys Val Lys Gly Thr Asn
    130                 135                 140

Glu Glu Met Val Phe Arg Gly Asn Val Asp Asn Asn Thr Pro Tyr Ala
145                 150                 155                 160

Asn Ser Phe Thr Pro Pro Ile Lys Ala Gln Tyr Val Arg Leu Tyr Pro
                165                 170                 175

Gln Ile Cys Arg Arg His Cys Thr Leu Arg Met Glu Leu Leu Gly Cys
            180                 185                 190

Glu Leu Ser Gly Cys
        195

<210>  43
<211>  1937
<212>  DNA
<213>  Mus musculus

<220>
<221>  CDS
<222>  (211)..(1050)

<400>  43
tgaggcttct cagcttcaga tgcaagtgag tgggtgtctc acagagaagc aaagagaaga      60

gaacaggaga aaggtgtttc ccttgactgc ggaaacttta taaagaaaac ttagcttctc     120

tggagcagtc agcgtcagag ttctgtcctt gacacctgag tctcctccac aaggctgtga     180

gaaggaaacc ctttcctggg gctgggtgcc atg cag cag ccc atg aat tac cca      234
                                 Met Gln Gln Pro Met Asn Tyr Pro
                                 1               5

tgt ccc cag atc ttc tgg gta gac agc agt gcc act tca tct tgg gct      282
Cys Pro Gln Ile Phe Trp Val Asp Ser Ser Ala Thr Ser Ser Trp Ala
    10                  15                  20

cct cca ggg tca gtt ttt ccc tgt cca tct tgt ggg cct aga ggg ccg      330
Pro Pro Gly Ser Val Phe Pro Cys Pro Ser Cys Gly Pro Arg Gly Pro
25                  30                  35                  40

gac caa agg aga ccg cca cct cca cca cca cct gtg tca cca cta cca      378
Asp Gln Arg Arg Pro Pro Pro Pro Pro Pro Pro Val Ser Pro Leu Pro
                45                  50                  55

ccg cca tca caa cca ctc cca ctg ccg cca ctg acc cct cta aag aag      426
Pro Pro Ser Gln Pro Leu Pro Leu Pro Pro Leu Thr Pro Leu Lys Lys
            60                  65                  70

aag gac cac aac aca aat ctg tgg cta ccg gtg gta ttt ttc atg gtt      474
Lys Asp His Asn Thr Asn Leu Trp Leu Pro Val Val Phe Phe Met Val
        75                  80                  85

ctg gtg gct ctg gtt gga atg gga tta gga atg tat cag ctc ttc cac      522
Leu Val Ala Leu Val Gly Met Gly Leu Gly Met Tyr Gln Leu Phe His
    90                  95                  100

ctg cag aag gaa ctg gca gaa ctc cgt gag ttc acc aac caa agc ctt      570
Leu Gln Lys Glu Leu Ala Glu Leu Arg Glu Phe Thr Asn Gln Ser Leu
105                 110                 115                 120

aaa gta tca tct ttt gaa aag caa ata gcc aac ccc agt aca ccc tct      618
Lys Val Ser Ser Phe Glu Lys Gln Ile Ala Asn Pro Ser Thr Pro Ser
                125                 130                 135

gaa aaa aaa gag ccg agg agt gtg gcc cat tta aca ggg aac ccc cac     666
Glu Lys Lys Glu Pro Arg Ser Val Ala His Leu Thr Gly Asn Pro His
            140                 145                 150

tca agg tcc atc cct ctg gaa tgg gaa gac aca tat gga acc gct ctg     714
Ser Arg Ser Ile Pro Leu Glu Trp Glu Asp Thr Tyr Gly Thr Ala Leu
        155                 160                 165

atc tct gga gtg aag tat aag aaa ggt ggc ctt gtg atc aac gaa act     762
Ile Ser Gly Val Lys Tyr Lys Lys Gly Gly Leu Val Ile Asn Glu Thr
    170                 175                 180

ggg ttg tac ttc gtg tat tcc aaa gta tac ttc cgg ggt cag tct tgc     810
Gly Leu Tyr Phe Val Tyr Ser Lys Val Tyr Phe Arg Gly Gln Ser Cys
185                 190                 195                 200

aac aac cag ccc cta aac cac aag gtc tat atg agg aac tct aag tat     858
Asn Asn Gln Pro Leu Asn His Lys Val Tyr Met Arg Asn Ser Lys Tyr
                205                 210                 215

cct gag gat ctg gtg cta atg gag gag aag agg ttg aac tac tgc act     906
Pro Glu Asp Leu Val Leu Met Glu Glu Lys Arg Leu Asn Tyr Cys Thr
            220                 225                 230

act gga cag ata tgg gcc cac agc agc tac ctg ggg gca gta ttc aat     954
Thr Gly Gln Ile Trp Ala His Ser Ser Tyr Leu Gly Ala Val Phe Asn
        235                 240                 245

ctt acc agt gct gac cat tta tat gtc aac ata tct caa ctc tct ctg     1002
Leu Thr Ser Ala Asp His Leu Tyr Val Asn Ile Ser Gln Leu Ser Leu
    250                 255                 260

atc aat ttt gag gaa tct aag acc ttt ttc ggc ttg tat aag ctt taa     1050
Ile Asn Phe Glu Glu Ser Lys Thr Phe Phe Gly Leu Tyr Lys Leu
265                 270                 275

aagaaaaagc attttaaaat gatctactat tctttatcat gggcaccagg aatattgtct     1110

tgaatgagag tcttcttaag acctattgag attaattaag actacatgag ccacaaagac     1170

ctcatgaccg caaggtccaa caggtcagct atccttcatt ttctcgaggt ccatggagtg     1230

gtccttaatg cctgcatcat gagccagatg gaaggaggtc tgtgactgag ggacataaag     1290

ctttgggctg ctgtgtgaca atgcagaggc acagagaaag aactgtctga tgttaaatgg     1350

ccaagagaat tttaaccatt gaagaagaca cctttacact cacttccagg gtgggtctac     1410

ttactacctc acagaggccg tttttgagac atagttgtgg tatgaatata caagggtgag     1470

aaaggaggct catttgactg ataagctaga gactgaaaaa aagacagtgt ctcattggca     1530

ccatctttac tgttacctaa tgttttctga gccgaccttt gatcctaacg gagaagtaag     1590

agggatgttt gaggcacaaa tcattctcta catagcatgc atacctccag tgcaatgatg     1650

tctgtgtgtt tgtatgtatg agagcaaaca gattctaagg agtcatataa ataaaatatg     1710

tacattatgg agtacatatt agaaacctgt tacatttgat gctagatatc tgaatgtttc     1770

ttggcaataa actctaatag tcttcaaaat cttttattat cagctactga tgctgttttt     1830

ctttaataca actagtattt atgctctgaa catcctaatg aggaaaagac aaataaaatt     1890

atgttataga atacagaaat gccttaagga catagacttt ggaaatc                    1937

<210>  44
<211>  279
<212>  PRT
<213>  Mus musculus

<400>  44

Met Gln Gln Pro Met Asn Tyr Pro Cys Pro Gln Ile Phe Trp Val Asp
1               5                   10                  15

Ser Ser Ala Thr Ser Ser Trp Ala Pro Pro Gly Ser Val Phe Pro Cys
            20                  25                  30

Pro Ser Cys Gly Pro Arg Gly Pro Asp Gln Arg Arg Pro Pro Pro Pro
        35                  40                  45

Pro Pro Pro Val Ser Pro Leu Pro Pro Pro Ser Gln Pro Leu Pro Leu
    50                  55                  60

Pro Pro Leu Thr Pro Leu Lys Lys Lys Asp His Asn Thr Asn Leu Trp
65                  70                  75                  80

Leu Pro Val Val Phe Phe Met Val Leu Val Ala Leu Val Gly Met Gly
                85                  90                  95

Leu Gly Met Tyr Gln Leu Phe His Leu Gln Lys Glu Leu Ala Glu Leu
            100                 105                 110

Arg Glu Phe Thr Asn Gln Ser Leu Lys Val Ser Ser Phe Glu Lys Gln
        115                 120                 125

Ile Ala Asn Pro Ser Thr Pro Ser Glu Lys Lys Glu Pro Arg Ser Val
    130                 135                 140

Ala His Leu Thr Gly Asn Pro His Ser Arg Ser Ile Pro Leu Glu Trp
145                 150                 155                 160

Glu Asp Thr Tyr Gly Thr Ala Leu Ile Ser Gly Val Lys Tyr Lys Lys
                165                 170                 175

Gly Gly Leu Val Ile Asn Glu Thr Gly Leu Tyr Phe Val Tyr Ser Lys
            180                 185                 190

Val Tyr Phe Arg Gly Gln Ser Cys Asn Asn Gln Pro Leu Asn His Lys
        195                 200                 205

Val Tyr Met Arg Asn Ser Lys Tyr Pro Glu Asp Leu Val Leu Met Glu
    210                 215                 220

Glu Lys Arg Leu Asn Tyr Cys Thr Thr Gly Gln Ile Trp Ala His Ser
225                 230                 235                 240

Ser Tyr Leu Gly Ala Val Phe Asn Leu Thr Ser Ala Asp His Leu Tyr
                245                 250                 255

Val Asn Ile Ser Gln Leu Ser Leu Ile Asn Phe Glu Glu Ser Lys Thr
            260                 265                 270

Phe Phe Gly Leu Tyr Lys Leu
        275

## Description

### TECHNICAL FIELD

The present invention relates to apoptosis-inducing agents, etc. More specifically, the present invention relates to apoptosis-inducing agents which induce apoptosis in various cells including tumor cells and other cells, etc.

### BACKGROUND ART

Apoptosis is one of the modes of cell death and particularly refers to cell death associated with nuclear destruction in cells. Molecular mechanisms previously known for apoptotic death in cancer cells are those induced thorough the following five pathways: calcium pathway, death signaling pathway, ceramide pathway, mitochondrial pathway and p53 apoptosis pathway (see, e.g., "Yoshiyuki Hashimoto, New Molecular Medicine of Apoptosis, Yodosha Co., Ltd., Japan, April 2001, pages 10-58").

Many substances are known to induce apoptosis, and anticancer agents such as mitomycin and taxol are used in clinical medicine. In addition to these artificial substances, apoptosis-inducing proteins such as FasL (Fas ligand) and TNFα can be found *in vivo.* These artificial and natural apoptosis-inducing substances can be used to induce the death of cells which are harmful to the human body, such as cancer cells. As described above, not only anticancer agents used in chemotherapy, but also FasL which is now being studied for its use in gene therapy relies on this principle. In chemotherapy, side effects on normal tissues serve as a hindrance to the therapy, and hence studies have now been conducted on gene therapy involving gene transfer into a lesion, with the expectation of producing an effect only on the lesion. However, cells that will actually receive gene transfer are usually limited to some of the cells present in a lesion. Moreover, diseased cells receiving gene transfer will then die and therapeutically effective proteins or the like will be no longer produced from these cells. Eventually, the effect of gene therapy is local in most cases. For this reason, residual diseased cells are required to repeatedly receive gene transfer. Moreover, cells receiving no gene during the fist gene transfer may be resistant to gene transfer and serve as a hindrance to the therapy.

### SUMMARY OF THE INVENTION

Under these circumstances, there has been a demand for the development of apoptosis-inducing agents capable of inducing apoptosis in various cells including tumor cells and other cells in a simple manner.

The present invention has been made in consideration of the above situation and aims to provide an apoptosis-inducing agent, a pharmaceutical composition for cancer treatment and so on, as shown below.
(1) An apoptosis-inducing agent, which comprises a peptide shown in (a) or (b) below, a derivative thereof, or a salt of the peptide or the derivative:
   (a) a peptide which comprises an amino acid sequence represented by the following formula (I):
      C-X-D-X-X-X-X-Y-X-C-X-C (SEQ ID NO: 1) (I)
      (wherein X represents any amino acid residue, C represents cysteine, D represents aspartic acid, and F represents phenylalanine); or
   (b) a peptide which comprises an amino acid sequence with deletion, substitution or addition of one or several amino acids in the amino acid sequence represented by formula (I) and which has apoptosis-inducing activity.

   In the inducing agent according to (1) above, the amino acid sequence represented by formula (I) may be, for example, the amino acid sequence shown in SEQ ID NO: 2 or SEQ ID NO: 3. Likewise, the peptide shown in (a) above may be, for example, a peptide comprising the amino acid sequence shown in SEQ ID NO: 5 or SEQ ID NO: 7.
(2) A method for inducing apoptosis, which comprises the step of treating a target cell or a target cell population with the inducing agent according to (1) above. In this method, the above step may be, for example, intended to contact the target cell or target cell population with the inducing agent.
(3) A pharmaceutical composition for cancer treatment, which comprises the inducing agent according to (1) above.

### BRIEF DESCRIPTION OF THE DRAWINGS

Figure 1 shows the results observed under a phase contrast microscope and a fluorescent microscope in Example 1.
Figure 2 shows the results of polyacrylamide gel electrophoresis (left panel) and the results measured for lactate dehydrogenase (LDH) activity (right panel) in Example 2.
Figure 3 shows the results observed under a fluorescent microscope in Example 3.
Figure 4 shows the results observed under a fluorescent microscope in Example 4.
Figure 5 shows the results observed under a fluorescent microscope in Example 5.
Figure 6 shows the results of polyacrylamide gel electrophoresis and silver staining in Example 6.
Figure 7 shows the results measured for LDH activity in Example 7.
Figure 8 shows the results observed under a phase contrast microscope and a fluorescent microscope in Example 8.
Figure 9 shows the results measured for LDH activity in Example 9.
Figure 10 shows the results measured for LDH activity in Example 10.
Figure 11 shows the results measured for LDH activity in Example 11.
Figure 12 shows the results of tumor size after each gene transfer, as measured over time (for 7 days: DayO to Day7) in Example 12. For measurement of tumor size, the long axis and short axis of each tumor were measured with a ruler and multiplied by each other. In the figure, the tumor size (vertical axis) is expressed as a relative value (mean ± SD), assuming that the value obtained at the time of gene transfer is set to 1.

### BEST MODES FOR CARRYING OUT THE INVENTION

The present invention will be described in more detail below. The scope of the present invention is not limited by the following description, and any embodiments other than those illustrated below may also be carried out with appropriate modifications without departing from the spirit of the invention.

It should be noted that this specification incorporates the specification of Japanese Patent Application No. 2009-194747 (filed on August 25, 2009) in its entirety, based on which the present application claims priority. Moreover, all publications cited herein, including prior art documents, patent gazettes and other patent documents, are incorporated herein by reference.

### 1. Apoptosis-inducing agent

As described above, the apoptosis-inducing agent of the present invention (hereinafter referred to as the inducing agent of the present invention) comprises a peptide shown in (a) below:
(a) a peptide which comprises an amino acid sequence represented by the following formula (I):
   C-X-D-X-X-X-X-Y-X-C-X-C (SEQ ID NO: 1) (I).

In the peptide shown in (a) above, the amino acid sequence of formula (I) is expressed with one letter codes of amino acids. For example, C, D and Y denote cysteine (Cys), aspartic acid (Asp) and tyrosine (Tyr), respectively. X represents any amino acid residue, which may generally be selected from 20 types of amino acid residues, i.e., A (alanine; Ala), R (arginine; Arg), D (aspartic acid; Asp), N (asparagine; Asn), C (cysteine; Cys), Q (glutamine; Gln), E (glutamic acid; Glu), G (glycine; Gly), H (histidine; His), I (isoleucine; Ile), L (leucine; Leu), K (lysine; Lys), M (methionine; Met), F (phenylalanine; Phe), P (proline; Pro), S (serine; Ser), T (threonine; Thr), W (tryptophan; Trp), Y (tyrosine; Tyr) and V (valine; Val). In this specification, other amino acid sequences may also be expressed with one letter codes, as in the case of formula (I).

Preferred examples of the above amino acid sequence of formula (I) include the following amino acid sequences shown in SEQ ID NO: 2 and SEQ ID NO: 3. Among them, more preferred is the amino acid sequence shown in SEQ ID NO: 2.
C-T-D-L-V-A-N-Y-S-C-E-C (SEQ ID NO: 2)
C-K-D-D-I-S-S-Y-E-C-W-C (SEQ ID NO: 3)

As used herein, the term "peptide" refers to a structure in which at least two amino acids are linked together via peptide bonds, including oligopeptides, polypeptides and so on. Moreover, a polypeptide formed into a certain three-dimensional structure is referred to as a protein, and such a protein also falls within the scope of the above "peptide" in the present invention. Thus, the peptide included in the inducing agent of the present invention may be any of an oligopeptide, a polypeptide and a protein.

Alternatively, the inducing agent of the present invention may comprise a peptide shown in (b) below, as described above:
(b) a peptide which comprises an amino acid sequence with deletion, substitution or addition of one or several amino acids in the amino acid sequence represented by formula (I) above and which has apoptosis-inducing activity.

Examples of the above "amino acid sequence with deletion, substitution or addition of one or several amino acids" include, but are not limited to, amino acid sequences with deletion, substitution or addition of about 1 to 5 amino acids, preferably about 1 to 2 amino acids. However, preferred for use in the present invention are those without deletion, substitution or addition of amino acid residues at positions 1, 3, 8, 10 and 12 (C, D, Y, C and C, respectively, in this order) in the amino acid sequence represented by formula (I) above, and/or those without deletion or addition of amino acid residues at positions 2, 4-7, 9 and 11 (each of which is X) in the amino acid sequence represented by formula (I) above. Introduction of mutations such as deletion, substitution or addition as described above may be accomplished by using a kit for mutation introduction based on site-directed mutagenesis, including GeneTailor^{™} Site-Directed Mutagenesis Systems (Invitrogen) and TaKaRa Site-Directed Mutagenesis Systems (e.g., Mutan-K, Mutan-Super Express Km; Takara Bio Inc., Japan). Further, whether or not a peptide has the above mutation (i.e., deletion, substitution or addition) can be confirmed by using various techniques for amino acid sequencing, as well as X-ray or NMR-based structural analysis, etc.

As used herein, the term "apoptosis-inducing activity" is intended to mean the ability to induce cell death associated with nuclear destruction in cells, and this activity can be measured, for example, by DNA ladder detection, chromatin staining, annexin V staining, etc.

The above peptide (a) or (b) included in the inducing agent of the present invention may be constructed from any number of amino acid residues as long as it is a peptide comprising the above amino acid sequence of formula (I) or a peptide comprising an amino acid sequence with deletion, substitution or addition as described above. The number of amino acid residues is preferably 12 or more, and may be 12 to 100, 12 to 50, or 12 to 30, by way of example.

The above peptide (a) or (b) may be either derived from a natural product or artificially obtained by chemical synthesis. Without being limited thereto, preferred are peptides derived from natural products because they often have no adverse effects (e.g., cytotoxicity) on any cells other than target cells of apoptosis induction.

Examples of peptides derived from natural products include naturally-occurring oligopeptides, polypeptides and proteins, or fragments thereof, etc. Such a peptide derived from a natural product may be obtained directly from the natural product by known collection and purification techniques or may be obtained by known gene recombination technology, in which a gene encoding this peptide is integrated into any of various expression vectors or the like and introduced into a cell to express the peptide, followed by known collection and purification techniques. Alternatively, such a peptide may be produced in a cell-free protein synthesis system using a commercially available kit (e.g., a reagent kit PROTEIOS^{™} (Toyobo Co., Ltd., Japan) or TNT^{™} System (Promega), a synthesizer PG-Mate^{™} (Toyobo Co., Ltd., Japan) or RTS (Roche Diagnostics)) and obtained by known collection and purification techniques. Peptides derived from natural products may be obtained in any way.

On the other hand, chemically synthesized peptides can be obtained by using known peptide synthesis techniques. Examples of synthesis techniques used for this purpose include azide method, acid chloride method, acid anhydride method, mixed acid anhydride method, DCC method, active ester method, carboimidazole method and oxidation-reduction method, etc. Moreover, their synthesis may be accomplished by applying either solid phase synthesis or liquid phase synthesis. A commercially available peptide synthesizer may also be used for this purpose. After synthesis reaction, the resulting peptides may be purified by using known purification techniques (e.g., chromatography) in combination.

As examples of a peptide derived from a natural product and falling within the above peptide (a) or (b), (i) developmentally regulated endothelial cell locus-1 (Del-1; developmentally endothelial locus-1) protein and a partial fragment thereof, as well as (ii) blood coagulation factor IX will be illustrated below.

Del-1 protein is an extracellular matrix protein having epidermal growth factor (EGF)-like domains and discoidin 1-like domains, which consists of the amino acid sequence shown in SEQ ID NO: 5. This Del-1 protein is known to have EGF1, EGF2 and EGF3 as EGF-like domains and have Discoidin1 and Discoidin2 as discoidin I-like domains. The inventors of the present invention have focused on EGF3 among the above various domains and have further focused on a region consisting of specific 12 amino acid residues: C-T-D-L-V-A-N-Y-S-C-E-C (SEQ ID NO: 2) in the amino acid sequence constituting EGF3 (SEQ ID NO: 7). As a result, the inventors have found that a peptide consisting of this specific region or comprising this specific region has high apoptosis-inducing activity. Namely, examples of a peptide derived from a natural product and falling within the above peptide (a) or (b) include the full-length Del-1 protein (SEQ ID NO: 5), a partial fragment consisting of EGF3 (SEQ ID NO: 7), and various partial fragments comprising EGF3 (SEQ ID NOs: 9, 11, 13, 15, 17 and 19 (see Table 1 below), as well as SEQ ID NO: 42). In particular, preferred for use as the above peptide (a) is the full-length Del-1 protein (SEQ ID NO: 5), a partial fragment consisting of EGF3 (a peptide constituting EGF3; SEQ ID NO: 7), or a fusion peptide between EGF3 and Discoidin1 (SEQ ID NO: 42). For details of the Del-1 protein and partial fragments thereof, reference may be made to the entire contents disclosed in WO2005/0001093. In addition to partial fragments having the amino acid sequences shown in Table 1 below, other partial fragments may also be included as embodiments of the present invention. By way of example, a partial fragment having an amino acid sequence modified to remove the signal peptide segment from "full-length Del-1" in Table 1 below is also included as an embodiment of the present invention.

**Table 1**

| Name of fragment or the like | Region* | Type | SEQ ID NO: |
|---|---|---|---|
| Full-length Del-1 | 619-2061 | DNA | 4 |
| | 1-480 | Protein | 5 |
| EGF3 | 985-1089 | DNA | 6 |
| | 123-157 | Protein | 7 |
| 4-1 | 619-1662 | DNA | 8 |
| | 1-348 | Protein | 9 |
| 4-15 | 619-1713 | DNA | 10 |
| | 1-365 | Protein | 11 |
| 4-14 | 619-1722 | DNA | 12 |
| | 1-368 | Protein | 13 |
| 4-13 | 619-1773 | DNA | 14 |
| | 1-385 | Protein | 15 |
| XY | 985-1662 | DNA | 16 |
| | 123-348 | Protein | 17 |
| XC | 985-1269 | DNA | 18 |
| | 123-217 | Protein | 19 |
| human XY | - | DNA | 20 |
| | - | Protein | 21 |

| | | | |
|---|---|---|---|
| * Regions in DNAs are indicated by nucleotide numbers, while regions in proteins are indicated by amino acid numbers. | | | |

It should be noted that nucleotide numbers and amino acid numbers used herein are corresponding numbers on the full-length Del-1 sequence.

Blood coagulation factor IX, which consists of the amino acid sequence shown in SEQ ID NO: 23, is a single-stranded glycoprotein having a Gla domain, an EGF domain and a protease domain in this order from the N-terminal end. The EGF domain of the factor IX is constituted by a sequence (SEQ ID NO: 25) consisting of amino acids at positions 90 to 123 in the amino acid sequence shown in SEQ ID NO: 23. The inventors of the present invention have focused on the fact that the amino acid sequence constituting the EGF domain of the factor IX contains a region consisting of specific 12 amino acid residues: C-K-D-D-I-S-S-Y-E-C-W-C (SEQ ID NO: 3), as in the case of EGF3 (EGF-like domain) in the Del-1 protein described above, and have found that a peptide comprising this region can increase the efficiency of gene transfer. Namely, examples of a peptide derived from a natural product and falling within the above peptide (a) or (b) include the full-length factor IX, as well as a peptide fragment consisting of the amino acid sequence shown in SEQ ID NO: 3 or various partial fragments comprising this fragment, and a partial fragment consisting of the EGF domain or various partial fragments comprising this EGF domain. It should be noted that the nucleotide sequence of DNA encoding the full-length factor IX is shown in SEQ ID NO: 22, while the nucleotide sequence of DNA encoding the EGF domain of the factor IX is shown in SEQ ID NO: 24.

The inducing agent of the present invention may comprise a derivative of the above peptide (a) or (b) together with or instead of the peptide. Such a derivative is intended to encompass all derivatives which can be prepared from the above peptide (a) or (b), and examples include derivatives whose constituent amino acids are partially replaced with unnatural amino acids, and/or derivatives whose constituent amino acids (mainly side chains thereof) are partially chemically modified.

The inducing agent of the present invention may comprise a salt of the above peptide (a) or (b) and/or a derivative of the peptide together with or instead of the peptide and/or the derivative. Such a salt is preferably a physiologically acceptable acid addition salt or basic salt. Examples of such an acid addition salt include salts with inorganic acids (e.g., hydrochloric acid, phosphoric acid, hydrobromic acid, sulfuric acid), as well as salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid). Examples of such a basic salt include salts with inorganic bases (e.g., sodium hydroxide, potassium hydroxide, ammonium hydroxide, magnesium hydroxide), as well as salts with organic bases (e.g., caffeine, piperidine, trimethylamine, pyridine).

The salt intended in the present invention can be prepared using a suitable acid such as hydrochloric acid or a suitable base such as sodium hydroxide. For example, the salt can be prepared by treatment in water or in a fluid containing an inert water-miscible organic solvent (e.g., methanol, ethanol or dioxane) according to a standard protocol.

Without being limited thereto, the inducing agent of the present invention may consist of the above peptide (a) or (b), a derivative thereof, or a salt of the peptide or the derivative, or may comprise the peptide, a derivative thereof, or a salt of the peptide or the derivative in combination with other components. Examples of other components include buffers such as PBS and Tris-HCl, as well as additives such as sodium azide and glycerol. In a case where the inducing agent of the present invention comprises other components, their content may be selected as appropriate within a range that does not significantly inhibit the ability to increase the efficiency of apoptosis induction provided by the above peptide, a derivative thereof, or a salt of the peptide or the derivative. More specifically, in the case of using the above peptide in a solution state, the peptide concentration is preferably, but not limited to, 0.3 ng/ml or higher, more preferably 0.3 to 5 ng/ml, even more preferably 0.3 to 2 ng/ml, still even more preferably 0.4 to 1.5 ng/ml, particularly preferably 0.6 to 1 ng/ml, and most preferably 0.8 to 1 ng/ml.

Target cells of apoptosis induced by the inducing agent of the present invention are not limited in any way and encompass various cells including tumor cells and other cells, with adherent cells being particularly preferred. Examples include Cos cells, CRL cells (human melanoma cell line), P5 cells (vascular endothelial cell line), KN cells (human squamous cell carcinoma cell line), etc. In the present invention, tumor cells are preferred for use as target cells of apoptosis induction, and specific examples of tumor cells preferably include CRL cells as mentioned above, as well as Cos cells, P5 cells, KN cells (human squamous cell carcinoma cell line), etc.

For induction of apoptosis in a target cell, it is not necessarily required to introduce the inducing agent of the present invention into the cell. When simply added from the outside of the cell, the inducing agent of the present invention can induce apoptosis in the cell. For this reason, apoptosis induction in various tumor cells can be achieved in a simple manner never before possible and in a wider range of cell types (cancer types). The inducing agent of the present invention is very useful in cancer treatment and/or studies thereof, by way of example.

The inducing agent of the present invention is **characterized in that** the above peptide (a) or (b) or the like can remain in the surrounding area (e.g., cancer lesion) of target cells where apoptosis is to be induced. Moreover, the above peptide (a) or (b) or the like is also characterized by having the ability to improve the efficiency of gene transfer into a cell. Thus, for example, if the inducing agent of the present invention is used for cancer treatment in combination with gene therapy, the inducing agent of the present invention (the above peptide (a) or (b) or the like) remaining in the lesion will exert an improving effect on the efficiency of gene transfer into residual diseased cells, e.g., when the diseased cells are required to repeatedly receive gene transfer. As a result, the inducing agent of the present invention allows gene therapy with high therapeutic effect.

The present invention can also provide a method for inducing apoptosis by using the inducing agent of the present invention. This method comprises the step of treating a target cell or a target cell population (tissue, organ) with the inducing agent of the present invention, and may further comprise any other non-limiting step(s). Treatment with the inducing agent of the present invention is intended to mean that the inducing agent of the present invention is contacted with a target cell or a target cell population (i.e., added to the cell or cell population and contacted with the outer surface thereof) or that the inducing agent of the present invention is introduced into a target cell. Above all, preferred is the former embodiment where the inducing agent is added. To introduce the inducing agent of the present invention into a target cell, the above peptide (a) or (b) serving as an active ingredient in the inducing agent of the present invention may be introduced in any way, either directly into the cell or in a state of DNA encoding the peptide (gene transfer). DNA introduction may be accomplished by using various known techniques for gene transfer, including liposome method (lipoplex method), polyplex method, peptide method, electroporation, and virus vector method.

### 2. DNA, recombinant vector, and transformant

### (1) DNA

The present invention also contemplates DNA which comprises a nucleotide sequence encoding an amino acid sequence constituting the above peptide (a) or (b). Without being limited thereto, such DNA may consist only of a nucleotide sequence encoding the above peptide (a) or (b) or may comprise this nucleotide sequence and additional known nucleotide sequences required for gene expression (e.g., transcription promoter, SD sequence, Kozak sequence, terminator). In the nucleotide sequence encoding the above peptide (a) or (b), the type of codon is not limited in any way. For example, after transcription, it is possible to use codons generally used in mammals (e.g., humans) or codons generally used in microorganisms (e.g., E. *coli* or yeast), plants or the like, which may be selected or designed as appropriate.

Moreover, the present invention also contemplates DNA which is hybridizable under stringent conditions with DNA consisting of a nucleotide sequence complementary to DNA comprising a nucleotide sequence encoding the above peptide (a) or (b) and which encodes a protein having apoptosis-inducing activity. As used herein, the term "stringent conditions" refers to, for example, a salt (sodium) concentration of 150 to 900 mM and a temperature of 55°C to 75°C, preferably a salt (sodium) concentration of 150 to 200 mM and a temperature of 60°C to 70°C.

### (2) Recombinant vector comprising DNA

The present invention also contemplates a recombinant vector carrying the above DNA of the present invention ligated to (inserted into) an appropriate vector. The DNA of the present invention may be inserted into any vector capable of replicating in a host, and examples of such a vector include plasmid DNAs, phage DNAs, viruses, etc.

Examples of plasmid DNAs include E. coli-derived plasmids, *Bacillus subtilis-*derived plasmids, yeast-derived plasmids and so on, while examples of phage DNAs include λ phage and so on. Likewise, examples of viruses include adenoviruses and retroviruses, etc.

In addition to a promoter and the DNA of the present invention, the recombinant vector of the present invention may further comprise a cis element (e.g., enhancer), a splicing signal, a poly(A) addition signal, a ribosome binding sequence (SD sequence), a selection marker gene, a reporter gene and so on, if desired. It should be noted that examples of a selection marker gene include the dihydrofolate reductase gene, the ampicillin resistance gene, the neomycin resistance gene, etc. Examples of a reporter gene include genes for green fluorescent protein (GFP) or mutants thereof (fluorescent proteins such as EGFP, BFP and YFP), luciferase, alkaline phosphatase, LacZ, etc.

### (3) Transformant

The present invention also contemplates a transformant obtainable by introducing the above recombinant vector of the present invention into a host such that a desired gene can be expressed. Any host may be used for this purpose as long as it is capable of expressing the DNA of the present invention, and it is possible to use bacteria, yeast and other microorganisms well known in the art, by way of example.

In the case of using a bacterium as a host, the recombinant vector of the present invention is not only autonomously replicable in the bacterium, but may also comprise a promoter, a ribosome binding sequence, the DNA of the present invention, and a transcription termination sequence. Examples of bacteria include *E. coli* (*Escherichia coli*) and so on. Examples of a promoter available for use include the lac promoter and so on. Techniques used for vector introduction into bacteria include various known introduction techniques, such as calcium ion method.

In the case of using yeast as a host, *Saccharomyces cerevisiae* or the like may be used, by way of example. In this case, any promoter may be used as long as it allows expression in yeast, and examples of such a promoter include the gall promoter and so on. Techniques used for vector introduction into yeast include, for example, electroporation, spheroplast method and so on.

### 3. Pharmaceutical composition

The inducing agent of the present invention (which can also be referred to as the peptide shown in (a) or (b) above) is useful as an active ingredient in pharmaceutical compositions.

Such pharmaceutical compositions are useful as those for cancer (tumor) treatment. In particular, because of having apoptosis-inducing activity in tumor cells or the like, the inducing agent of the present invention is preferred for use in tumor treatment. Namely, the inducing agent of the present invention is useful as an active ingredient in therapeutic agents for tumors.

Moreover, the inducing agent of the present invention can also be combined with existing antitumor agents (therapeutic agents for tumors) for use as active ingredients in pharmaceutical compositions for cancer treatment. In this case, the inducing agent of the present invention can eventually enhance the antitumor effect (antitumor activity) of the existing antitumor agents through its apoptosis-inducing activity. Thus, the present invention enables the provision of an enhancer comprising the inducing agent of the present invention for use in enhancing the antitumor effect of an antitumor agent, and a method for enhancing the antitumor effect of an antitumor agent by using the inducing agent of the present invention. Such an antitumor agent includes all known ones, as exemplified by, but not limited to, FasL (Fas ligand), TNFα, mitomycin and taxol, etc.

The pharmaceutical composition of the present invention is preferably provided in the form of a pharmaceutical composition comprising the inducing agent of the present invention as an active ingredient and further comprising a pharmaceutically acceptable carrier.

Specific examples of target cancer (tumor) to be treated by the pharmaceutical composition of the present invention include lung cancer (e.g., small cell lung cancer), large bowel cancer, breast cancer, liver cancer, kidney cancer, ovarian cancer, neuroendocrine tumor, neuroblastoma, glioma, neurofibromatosis type 1, gastric cancer, large bowel cancer, pancreatic cancer, bladder cancer, skin cancer, etc., with human cancers (tumors) of these types being preferred.

The term "pharmaceutically acceptable carrier" is intended to include excipients, diluents, extenders, disintegrants, stabilizers, preservatives, buffering agents, emulsifiers, aromatics, coloring agents, sweeteners, thickeners, correctives, solubilizers, as well as other additives, etc. One or more such carriers may be used to prepare pharmaceutical compositions in the form of injections, solutions, capsules, suspensions, emulsions or syrups, etc. These pharmaceutical compositions may be administered orally or parenterally. Other dosage forms for parenteral administration include, for example, injections which comprise one or more active agents and are formulated in a routine manner. In the case of injections, they may be prepared by being dissolved or suspended in a pharmaceutically acceptable carrier such as physiological saline or commercially available injectable distilled water. In a case where the inducing agent of the present invention (the peptide shown in (a) or (b) above) is administered *in vivo,* a colloidal dispersion system may also be used. Such a colloidal dispersion system can be expected to provide some effects, such as increased *in vivo* stability of the above peptide and/or efficient delivery of a compound to a specific organ, tissue or cell. The colloidal dispersion system is not limited in any way as long as it is commonly used, and examples include polyethylene glycol, polymer complexes, polymer assemblies, nanocapsules, microspheres, beads, as well as lipid-based dispersion systems including oil-in-water emulsions, micelles, mixed micelles and liposomes. Preferred are liposomes or artificial membrane vesicles which allow efficient delivery of a compound to a specific organ, tissue or cell.

The dose of the pharmaceutical composition of the present invention may vary depending on the age, sex, body weight and symptom of a patient, the intended therapeutic effect, the mode of administration, the time of treatment, or the type of the inducing agent of the present invention (the peptide shown in (a) or (b) above) to be contained in the pharmaceutical composition, etc. In general, the pharmaceutical composition of the present invention may be administered, but not limited to, at a single dose ranging from 100 µg to 5000 mg per adult.

For example, when administered in the form of injections, the pharmaceutical composition of the present invention may be administered at a single dose of 100 µg to 100 mg per kg body weight of a human patient on an average of one to several times a day. The mode of administration includes intravenous injection, subcutaneous injection, intracutaneous injection, intramuscular injection or intraperitoneal injection, with intravenous injection being preferred. In some cases, injections may be prepared as non-aqueous dilutions (e.g., polyethylene glycol, vegetable oils such as olive oil, alcohols such as ethanol), suspensions or emulsions. Such injections may be sterilized, for example, by being filtered through a filter or mixed with a disinfectant. Injections may be prepared in reconstitutable form. Namely, injections may be converted into sterile solid compositions by lyophilization or other techniques, and the resulting solid compositions may be dissolved in sterile injectable distilled water or other solvents before use.

It should be noted that the present invention also provides the use of the above inducing agent of the present invention (or the peptide shown in (a) or (b) above) for the manufacture of a medicament (drug) for cancer (tumor) treatment. Moreover, the present invention also provides the above inducing agent of the present invention (or the peptide shown in (a) or (b) above) for use in cancer (tumor) treatment.

Further, the present invention provides a method for cancer (tumor) treatment, **characterized in that** the above inducing agent of the present invention (or the peptide shown in (a) or (b) above) is used (i.e., administered to a patient), and also provides the use of the above inducing agent of the present invention (or the peptide shown in (a) or (b) above) for cancer (tumor) treatment.

### 4. Kit for apoptosis induction

The present invention also provides a kit for apoptosis induction, which comprises the inducing agent of the present invention as a constituent member. The kit of the present invention can be used for simple and effective induction of apoptosis in various cells including tumor cells and other cells, and is therefore significantly useful not only in the field of cancer treatment as described above, but also in the fields of various experiments, researches, etc.

### EXAMPLES

The present invention will be further described in more detail by way of the following examples, which are not intended to limit the scope of the present invention.

### [Example 1]

### Apoptosis induction by Del-1 (1)

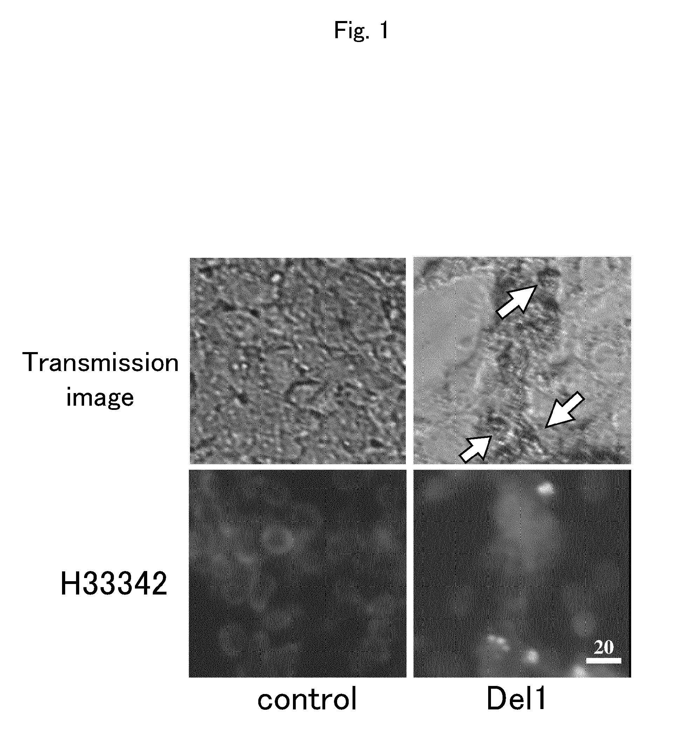

cDNA for full-length Del-1 (SEQ ID NO: 4) was integrated into an expression vector, pcDNA3 (Invitrogen), and then introduced into cultured Cos cells. As a control, pcDNA3 free from the cDNA for full-length Del-1 was used. At 24 hours after gene transfer, the cells were stained with H33342 (Hoechst) for cell nuclear staining and observed under a phase contrast microscope (transmission image) and a fluorescent microscope (H33342). The results obtained are shown in Figure 1.

As indicated with arrows in the figure, in the cultured Cos cells receiving gene transfer, some cells were being released from the bottom surface of the culture vessel, as can be seen from their transmission image under the phase contrast microscope, and their nuclei were strongly stained with H33342, thus indicating that their chromosomes were aggregated.

### [Example 2]

### Apoptosis induction by Del-1 (2)

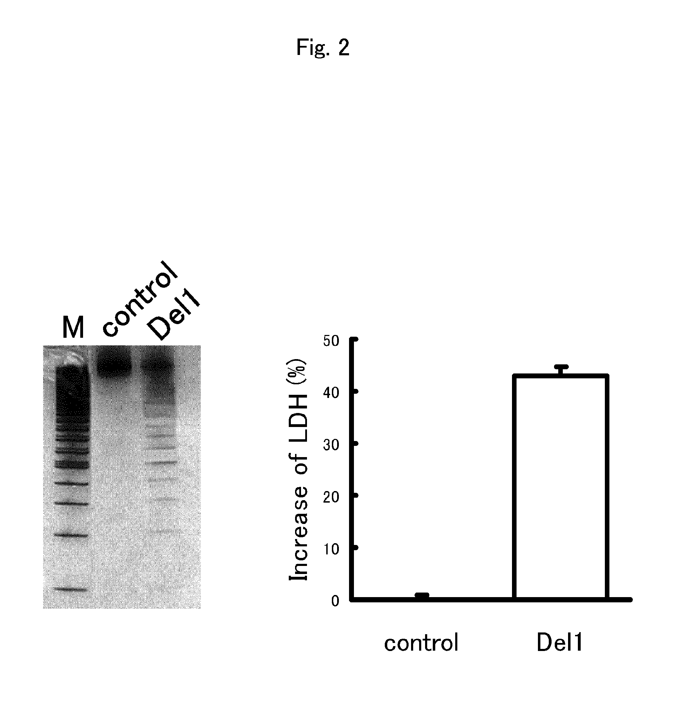

cDNA for full-length Del-1 (SEQ ID NO: 4) was integrated into an expression vector, pcDNA3 (Invitrogen), and then introduced into cultured Cos cells. As a control, pcDNA3 free from the cDNA for full-length Del-1 was used. For normalization of the gene transfer rate, DNA carrying the LacZ gene was also introduced simultaneously. The results obtained are shown in Figure 2 (left and right panels).

Left panel: Chromosomal DNAs were taken from the cells and electrophoresed on a polyacrylamide gel, followed by silver staining. A ladder pattern indicative of apoptosis was observed in a sample of the cells forced to express Del-1.

Right panel: Culture supernatants were measured for lactate dehydrogenase (LDH) activity. LDH, which is an enzyme generally found in the cytoplasm, was found to leak into the culture supernatant upon cell death.

### [Example 3]

### Apoptosis induction by Del-1 (3)

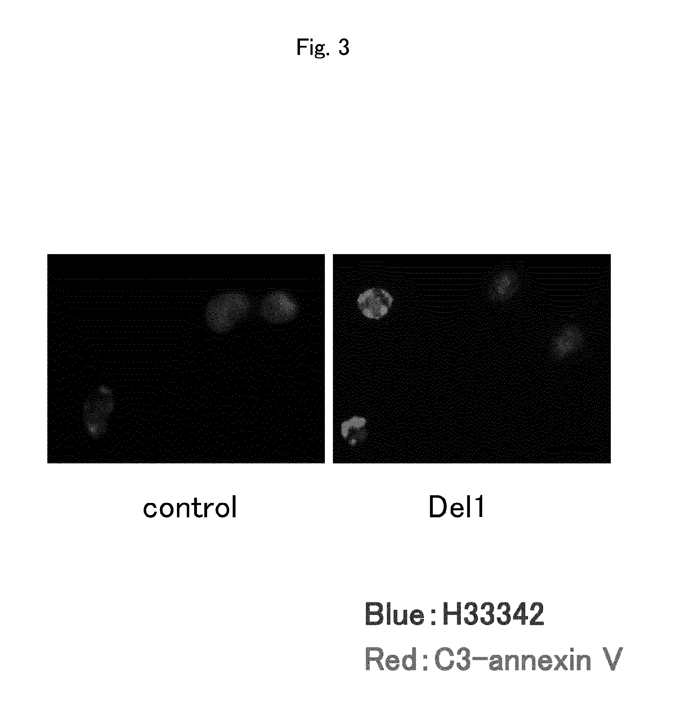

cDNA for full-length Del-1 (SEQ ID NO: 4) was integrated into an expression vector and then introduced into cultured Cos cells. At 24 hours after gene transfer, the cells were stained with H33342 and with C3-annexin V (annexin V conjugated with the fluorescent dye C3) and then observed under a fluorescent microscope. The results obtained are shown in Figure 3.

The cell membrane of cells whose chromosomes were found to aggregate, as analyzed by H33342 staining, was stained with C3-annexin V, indicating that these cells underwent apoptosis. It should be noted that annexin V is capable of binding to phosphatidylserine, a component of cell membranes, while phosphatidylserine is known to leak (to be localized) outside of the cell membrane when the cells undergo apoptosis.

### [Example 4]

### Apoptosis induction by Del-1 (4)

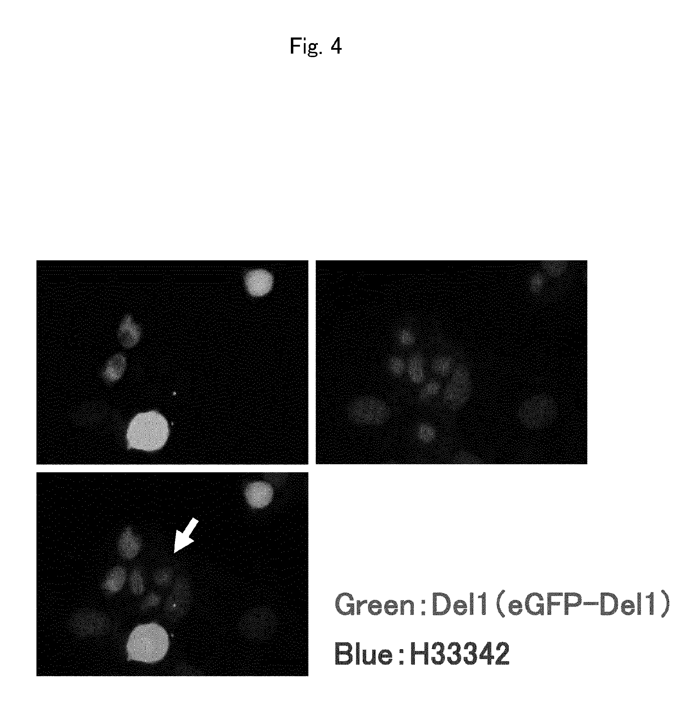

An expression vector designed to carry cDNA encoding eGFP-Del-1 (a fusion protein between eGFP and full-length Del-1) was introduced into cultured Cos cells. At 24 hours after gene transfer, the cells were fixed and stained with H33342. The results obtained are shown in Figure 4.

The cells receiving Del-1 gene transfer (i.e., the cells expressing the above fusion protein) were observed in green by the action of eGFP. These cells were found to undergo apoptosis because they showed chromosomal aggregation. Although the three cells indicated with the arrow in the figure showed no green fluorescence and did not express Del-1 (i.e., not receive Del-1 gene transfer), they showed chromosomal aggregation. This would mean that Del-1 secreted from the cells receiving Del-1 gene transfer would also act on their adjacent cells to thereby induce apoptosis in these cells.

### [Example 5]

### Apoptosis induction by Del-1 (5)

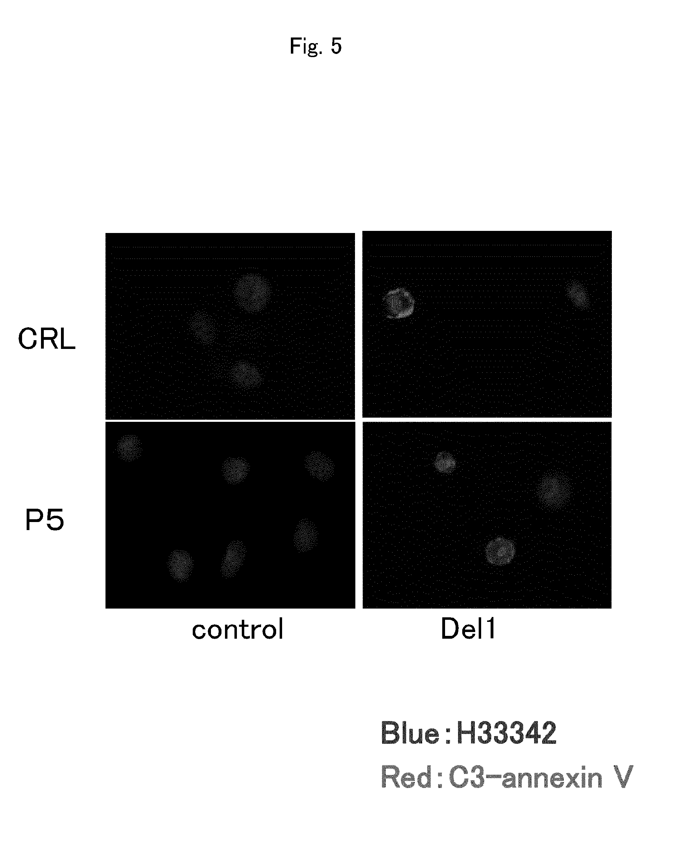

cDNA for full-length Del-1 (SEQ ID NO: 4) was integrated into an alkaline phosphatase (AP) expression vector (APtag4) and then introduced into cultured Cos cells. As a control, an AP expression vector free from the cDNA for full-length Del-1 was used. The resulting culture supernatant was measured for AP activity, normalized for AP fusion protein concentration and then added to a culture of CRL cells (human melanoma cells) or P5 cells (vascular endothelial cell line) (instead of using direct gene transfer, Cos cells were allowed to produce a recombinant protein and the resulting conditioned medium was used, because CRL and P5 were low in the efficiency of gene transfer). At 24 hours after addition, the cells were stained with H33342 and with C3-annexin V for observation. The results obtained are shown in Figure 5.

The cell membrane of cells whose chromosomes were found to aggregate, as analyzed by H33342 staining, was stained with C3-annexin V, indicating that these cells underwent apoptosis.

### [Example 6]

### Apoptosis induction by Del-1 (6)

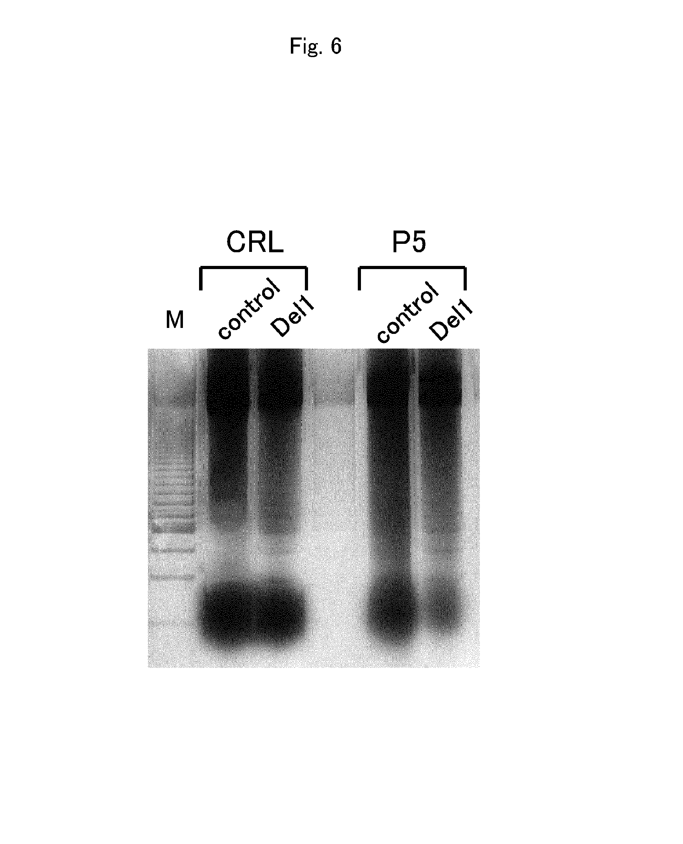

cDNA for full-length Del-1 (SEQ ID NO: 4) was integrated into an AP expression vector (APtag4) and then introduced into cultured Cos cells. As a control, an AP expression vector free from the cDNA for full-length Del-1 was used. The resulting culture supernatant was measured for AP activity, normalized for AP fusion protein concentration and then added to a culture of CRL cells (human melanoma cells) or P5 cells (vascular endothelial cell line) (instead of using direct gene transfer, Cos cells were allowed to produce a recombinant protein and the resulting conditioned medium was used, because CRL and P5 were low in the efficiency of gene transfer). At 24 hours after addition, chromosomal DNAs were taken from the cells and electrophoresed on a polyacrylamide gel, followed by silver staining. The results obtained are shown in Figure 6.

A ladder pattern indicative of apoptosis was observed in a sample of the cells forced to express Del-1.

### [Example 7]

### Active center in apoptosis induction (1)

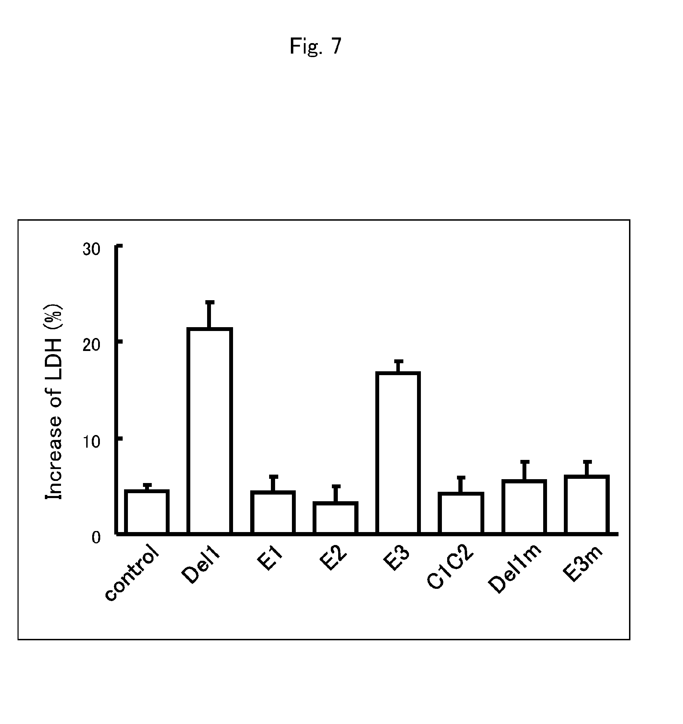

cDNA for full-length Del-1 (SEQ ID NO: 4) was integrated into an AP expression vector (APtag4) and then introduced into cultured Cos cells. Likewise, cDNA for a part of Del-1 was also integrated into APtag4 and then introduced into cultured Cos cells. As a control, an AP expression vector free from the above cDNA for full-length Del-1 or a part thereof was used. It should be noted that the members each used as a part of Del-1 were EGF-like domains in the full-length Del-1 protein, i.e., EGF1 (E1; cDNA: SEQ ID NO: 26, amino acid sequence: SEQ ID NO: 27), EGF2 (E2; cDNA: SEQ ID NO: 28, amino acid sequence: SEQ ID NO: 29) and EGF3 (E3; cDNA: SEQ ID NO: 6, amino acid sequence: SEQ ID NO: 7), a fusion protein between discoidin 1-like domains, i.e., Discoidin1 and Discoidin2 (ClC2; cDNA: SEQ ID NO: 30, amino acid sequence: SEQ ID NO: 31), an amino acid substitution (D 136E) mutant of the above full-length Del-1 (Del1m; cDNA: SEQ ID NO: 32, amino acid sequence: SEQ ID NO: 33), as well as an amino acid substitution (D136E) mutant of E3, i.e., E3 D136E (E3m; cDNA: SEQ ID NO: 34, amino acid sequence: SEQ ID NO: 35). It should be noted that the expression "136" in "D136E" which represents an embodiment of amino acid substitution in E3m denotes the position of the 136th amino acid residue in the amino acid sequence of full-length Del-1 (SEQ ID NO: 5), which corresponds to the position of the 14th amino acid residue in the amino acid sequence of E3 (SEQ ID NO: 7). For normalization of the gene transfer rate, DNA carrying the LacZ gene was also introduced simultaneously. At 24 hours after gene transfer, the medium in each case was replaced with fresh culture medium and, after further one hour, the supernatant was measured for LDH activity. A cell lysate was prepared for each case and measured for LacZ activity. Assuming that the LacZ activity of the cells transformed with cDNA for full-length Del-1 was set to 1, increases in LDH activity were normalized. The results obtained are shown in Figure 7.

The supernatant of the cells transformed with cDNA for E3 was found to show an increase in LDH activity similar to that of the cells transformed with cDNA for full-length Del-1. This effect was lost by D136E mutation, as can be seen from the results obtained for the cells transformed with cDNA for E3m.

### [Example 8]

### Active center in apoptosis induction (2)

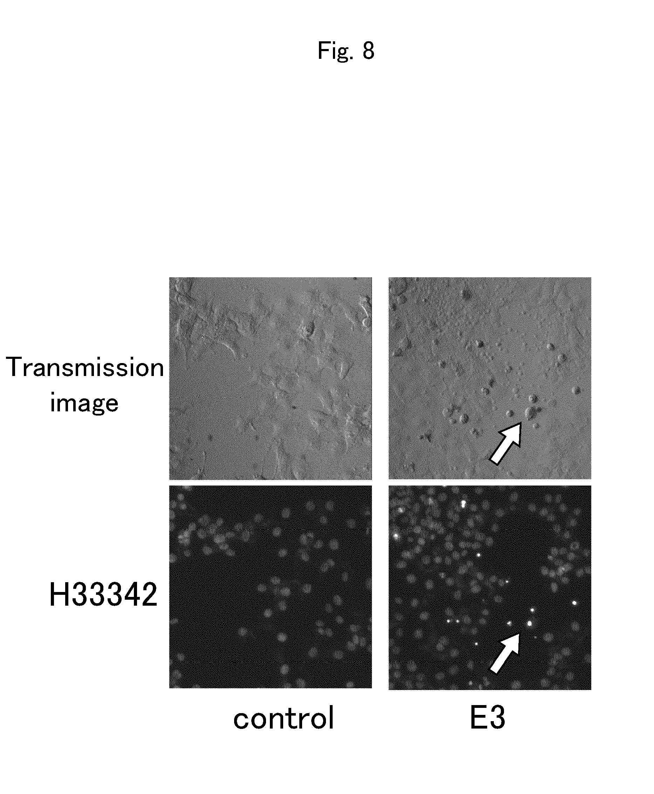

To cultured Cos cells, a recombinant E3 protein produced from transformed E. *coli* was added. At 24 hours after addition, the cells were stained with H33342 and observed under a phase contrast microscope and a fluorescent microscope. The results obtained are shown in Figure 8.

As indicated with arrows in the figure, in the cultured Cos cells after addition of the recombinant protein, some cells were being released from the bottom surface of the culture vessel, as can be seen from their transmission image under the phase contrast microscope, and their nuclei were strongly stained with H33342, thus indicating that their chromosomes were aggregated.

### [Example 9]

### Consensus sequence

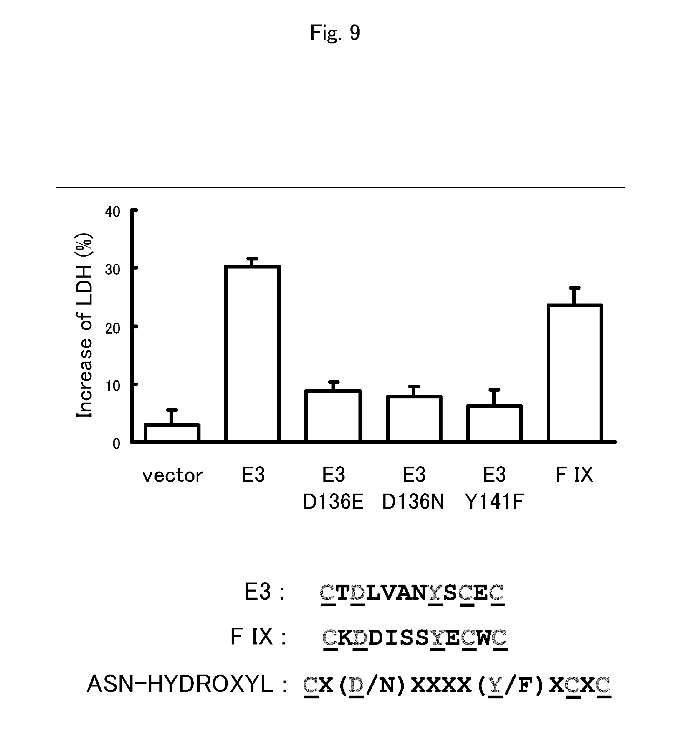

cDNA for E3 (a part of Del-1) or an amino acid substitution mutant thereof was integrated into an AP expression vector (APtag4) and then introduced into cultured Cos cells. Likewise, cDNA for the EGF domain of blood coagulation factor IX (F IX; cDNA: SEQ ID NO: 24, amino acid sequence: SEQ ID NO: 25) was also integrated into APtag4 and then introduced into cultured Cos cells. As a control, an AP expression vector free from the above cDNAs for E3 and so on was used. It should be noted that the amino acid substitution mutants of E3 used for the above purpose were E3 D136E (cDNA: SEQ ID NO: 34, amino acid sequence: SEQ ID NO: 35), E3 D136N (cDNA: SEQ ID NO: 36, amino acid sequence: SEQ ID NO: 37) and E3 Y141F (cDNA: SEQ ID NO: 38, amino acid sequence: SEQ ID NO: 39). It should be noted that the expressions "136" and "141" in "D136E", "D136N" and "Y141F" which represent embodiments of amino acid substitution in the E3 mutants denote the positions of the 136th and 141st amino acid residues, respectively, in the amino acid sequence of full-length Del-1 (SEQ ID NO: 5), which correspond to the positions of the 14th and 19th amino acid residues, respectively, in the amino acid sequence of E3 (SEQ ID NO: 7). For normalization of the gene transfer rate, DNA carrying the LacZ gene was also introduced simultaneously. At 4 hours after gene transfer, the medium in each case was replaced with fresh culture medium and, after further 24 hours, the supernatant was measured for LDH activity. A cell lysate was prepared for each case and measured for LacZ activity. Assuming that the LacZ activity of the cells transformed with E3 (wild-type) was set to 1, increases in LDH activity were normalized. The results obtained are shown in Figure 9.

The LDH activity was reduced in all cases transformed with the E3 mutants. This result indicated that the LDH activity was reduced by mutations in amino acid residues (D and Y in this example) corresponding to the specific amino acid residues (C, D, Y, C and C (amino acid residues at positions 1, 3, 8, 10 and 12 in SEQ ID NO: 40)) in the amino acid sequence (C-X-[D/N]-X-X-X-X-[Y/F]-X-C-X-C (SEQ ID NO: 40)) constituting the sequence for hydroxylation of asparagine residues (ASN-HYDROXYL). In contrast, the cells transformed with F IX (which has no amino acid residue in common with E3, except for the above specific amino acid residues) were found to have high LDH activity, as in the case of the E3-transformed cells.

### [Example 10]

### Concentration dependence

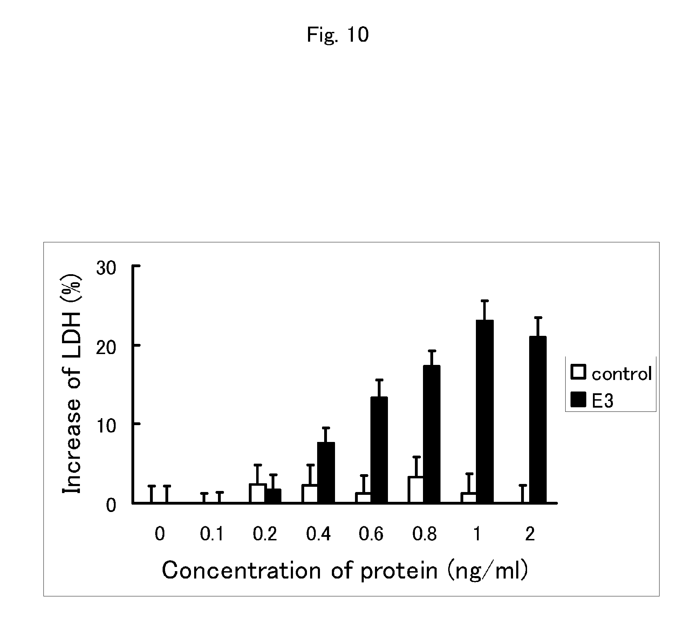

A recombinant E3 protein produced from transformed E. *coli* was added at different concentrations (0 to 2.0 ng/ml) to cultured Cos cells. At 24 hours after addition, the culture supernatants were each measured for LDH activity. The results obtained are shown in Figure 10.

E3 protein concentrations of 0.4 ng/ml or higher were found to be effective for apoptosis induction. It should be noted that the effect on apoptosis induction remained unchanged at E3 protein concentrations higher than 1 ng/ml.

### [Example 11]

### Effectiveness of E3 in various cells

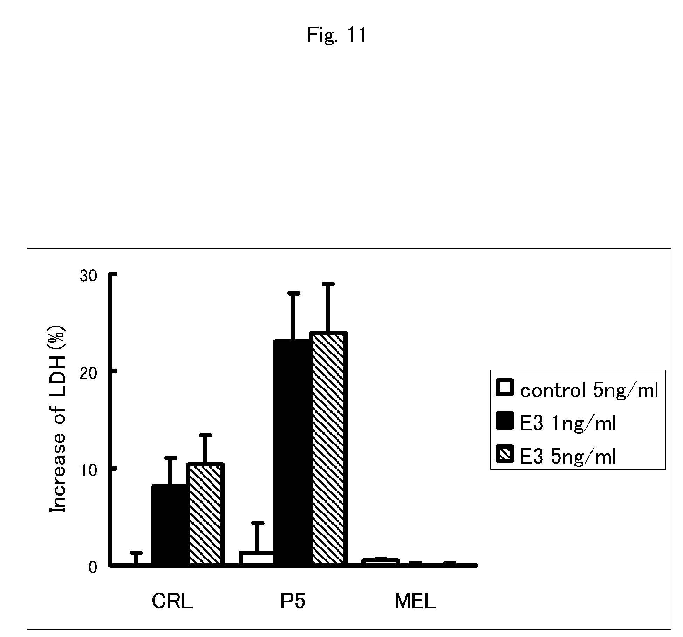

A recombinant E3 protein produced from transformed E. *coli* was added at a concentration of 1 ng/ml or 5 ng/ml to cultured Cos cells. At 24 hours after addition, the culture supernatants were each measured for LDH activity. The results obtained are shown in Figure 11.

The E3 protein showed cytotoxic activity (apoptosis induction) against CRL cells (human melanoma cells) and P5 cells (vascular endothelial cell line), which are adherent cells, whereas the E3 protein showed no cytotoxic activity against MEL cells (mouse leukemia cells), which are floating cells.

### [Example 12]

### Therapeutic effect on tumor provided by EGF3 domain-containing peptide

Using tumor-transplanted model mice, a peptide containing the EGF3 domain, which is a part of Del-1, was studied for its therapeutic effect on tumor. More specifically, this peptide was verified as to whether its combined use with the FasL (Fas ligand) protein, whose antitumor effect has already been established, would enhance the antitumor effect when compared to FasL alone.

First, cDNAs shown in (A) and (B) below were each integrated into an expression vector, pcDNA3D (Invitrogen), and then cloned to prepare two recombinant DNAs.
(A) cDNA for full-length mouse FasL (cDNA (GenBank accession No. NM_010177): SEQ ID NO: 43, amino acid sequence (GenBank accession No. NP_034307): SEQ ID NO: 44)
(B) cDNA in (A) above and cDNA for a fusion peptide between EGF3 domain and Discoidin1 (E3C1; cDNA: SEQ ID NO: 41, amino acid sequence: SEQ ID NO: 42) (two cDNAs in total)

It should be noted that E3C1 is a peptide consisting of amino acids at positions 123 to 319 of the amino acid sequence for full-length mouse Del-1 (SEQ ID NO: 5) (i.e., a peptide encoded by the nucleotide sequence located at positions 985 to 1575 of the nucleotide sequence for full-length mouse Del-1 (SEQ ID NO: 4)).

Then, 5 × 10⁶ SCCKN cells (human oral squamous cell carcinoma-derived cell line) were injected under the dorsal skin of nude mice at 5 weeks of age. The mice were then measured over time for their tumor size. At the time when the tumor size exceeded 10 mm × 10 mm, the mice were intratumorally injected (gene transfer) with the two recombinant DNAs prepared above to thereby express each cDNA in the tumor cells. During this gene transfer, *in vivo* jet-PEI (Polyplus Transfection) was used as a gene transfer reagent and the amount of DNA to be introduced was set to 10 µg. Four mice were used for introduction of the recombinant DNA containing the cDNA shown in (A) above, while five mice were used for introduction of the recombinant DNA containing the cDNAs shown in (B) above.

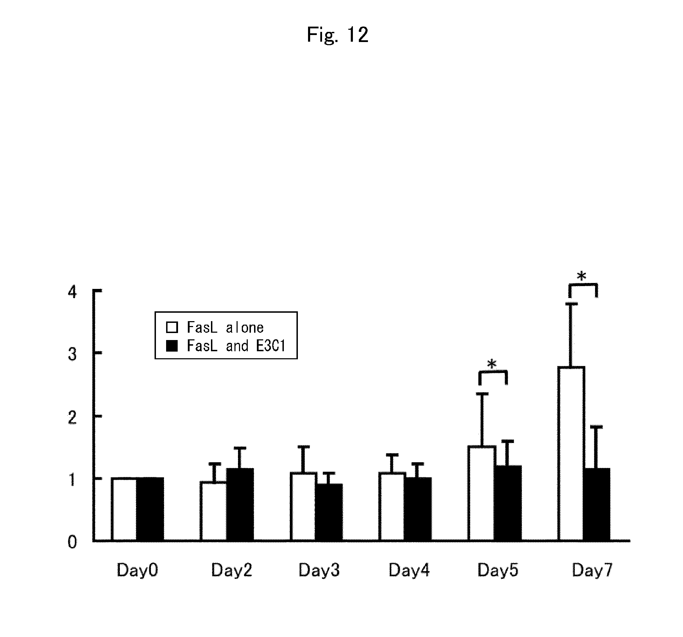

The tumor size after gene transfer was measured over time (for 7 days: Day0 to Day7), and the results obtained are shown in Figure 12. Introduction together with the cDNA for E3C1 provided a higher antitumor effect, thus indicating that E3C1 had an enhancing effect on the antitumor effect of FasL.

### INDUSTRIAL APPLICABILITY

The present invention enables the provision of apoptosis-inducing agents which induce apoptosis in various cells including tumor cells and other cells, etc.

The apoptosis-inducing agents of the present invention can induce apoptosis in a wide range of cell types in a simple manner, and also effectively induce apoptosis in tumor cells. Thus, the apoptosis-inducing agents of the present invention are very useful in that they can be effectively used, e.g., for pharmaceutical compositions or methods for cancer treatment.

Moreover, the apoptosis-inducing agents of the present invention are also advantageous in that they remain in the surrounding area (e.g., lesion) of target cells and are capable of improving the efficiency of gene transfer in the subsequent gene therapy, etc. Thus, the apoptosis-inducing agents of the present invention are very useful in that they can provide, for example, pharmaceutical compositions or methods for cancer treatment with high therapeutic effect when used in combination with agents for gene therapy.

### Sequence Listing Free Text

SEQ ID NO: 1: Peptide
SEQ ID NO: 1: Xaa at locations 2, 4, 5, 6, 7, 9 and 11 represents any amino acid residue.
SEQ ID NO: 40: Peptide
SEQ ID NO: 40: Xaa at locations 2, 4, 5, 6, 7, 9 and 11 represents any amino acid residue.
SEQ ID NO: 40: Xaa at location 3 represents aspartic acid or asparagine.
SEQ ID NO: 40: Xaa at location 8 represents tyrosine or phenylalanine.

## Claims

1. An apoptosis-inducing agent, which comprises a peptide shown in (a) or (b) below, a derivative thereof, or a salt of the peptide or the derivative:
(a) a peptide which comprises an amino acid sequence represented by the following formula (I):
C-X-D-X-X-X-X-Y-X-C-X-C (I)
(wherein X represents any amino acid residue, C represents cysteine, D represents aspartic acid, and F represents phenylalanine); or
(b) a peptide which comprises an amino acid sequence with deletion, substitution or addition of one or several amino acids in the amino acid sequence represented by formula (I) and which has apoptosis-inducing activity.

2. The inducing agent according to claim 1, wherein the amino acid sequence represented by formula (I) is the amino acid sequence shown in SEQ ID NO: 2 or SEQ ID NO: 3.

3. The inducing agent according to claim 1 or 2, wherein the peptide shown in (a) is a peptide comprising the amino acid sequence shown in SEQ ID NO: 5 or SEQ ID NO: 7.

4. A method for inducing apoptosis, which comprises the step of treating a target cell or a target cell population with the inducing agent according to any one of claims 1 to 3.

5. The method according to claim 4, wherein the step is intended to contact the target cell or target cell population with the inducing agent.

6. A pharmaceutical composition for cancer treatment, which comprises the inducing agent according to any one of claims 1 to 3.
